# EUROPEAN PATENT APPLICATION

(11) **EP 3 957 734 A1**
(43) Date of publication of application: **23.02.2022**
(21) Application number: 20791867.3
(22) Date of filing: 20.04.2020
(51) Int. Cl.: C12N 15/10, C12N 9/22, C12N 15/113, C12N 15/86, C12N 15/63, A61K 48/00, A61K 31/7088, A61P 31/20

(54) **COMPOSITION AND METHOD FOR INHIBITING PROLIFERATION OF HEPATITIS B VIRUS**

(30) Priority: 18.04.2019 US 201962835628 P
(71) Applicant: Toolgen Incorporated, Seoul 08501 (KR); Handong Global University Industry-Academic Cooperation Foundation, Pohang-si, Gyeongsangbuk-do 37554 (KR); Konkuk University Glocal Industry-Academic Collaboration Foundation, Chungju-si, Chungcheongbuk-do 27478 (KR)
(72) Inventor: LEE, Jae Young, Seoul 06721 (KR); LEE, Kyu Jun, Seoul 08714 (KR); LEE, Jung Min, Pohang-si Gyeongsangbuk-do 37669 (KR); KIM, Kyun Hwan, Seoul 05834 (KR); WON, Ju Hee, Suwon-si Gyeonggi-do 16421 (KR)
(74) Representative: dompatent von Kreisler Selting Werner - Partnerschaft von Patent- und Rechtsanwälten mbB
(86) International application number: PCT/KR2020/005203
(87) International publication number: WO 2020/214003

(57) **Abstract**

The present application relates to a composition and method for inhibiting Hepatitis B virus (HBV) proliferation.

## Description

### [Technical Field]

The present application relates to a method for inhibiting the proliferation of various hepatitis B virus genotypes.

The present application relates to a composition for inhibiting the proliferation of various hepatitis B virus genotypes.

The present application relates to various uses of a composition for inhibiting the proliferation of various hepatitis B virus genotypes.

### [Background Art]

Hepatitis B is an inflammation generated by the hepatitis B virus (herein referred to as HBV).

HBV infection not only has a high incidence worldwide, but can also occur in anyone regardless of age or race, and there are various routes of infection.

Hepatitis B therapeutics developed so far only improve symptoms for some of the various HBV genotypes, and thus hepatitis B often recurs.

In addition, various documents (Patent No. EP2014830911; World J Gastroenterol. 2015 Aug 28; 21(32): 9554-9565.) which obtained through research on the inhibition of HBV proliferation using a CRISPR/Cas system to reduce HBV activity also show that an HBV inhibitory effect depends on a target region, and varies for each of the HBV genotypes.

As such, in addition to efforts to reduce the recurrence of HBV, there is still a need for inhibition of the proliferation of various HBV genotypes.

### [Disclosure]

### [Technical Problem]

One aspect of the present application is to providing a method for inhibiting HBV proliferation by targeting conserved regions of various HBV genotypes.

Another aspect of the present application is to providing a composition for targeting conserved regions of various HBV genotypes.

Still another aspect of the present application is to providing conserved sequences in conserved regions of various HBV genotypes.

### [Technical Solution]

To solve the above-described problems, the present application may provide a method for inhibiting HBV proliferation. Here, the HBV includes at least two HBV genotypes selected from HBV genotype A, HBV genotype B, HBV genotype C, HBV genotype D, HBV genotype E, HBV genotype F and HBV genotype G,

The method may be provided that it comprises:
introducing into a subject a composition comprising:
   a) a guide nucleic acid, or a nucleic acid sequence encoding the same; and
   b) a Cas protein, or a nucleic acid sequence encoding the same;
wherein the guide nucleic acid selected from SEQ ID NO.51, 52, 56, 57, 69, 70, 73, 74, 75, 76, 80, 85, 87 and 89. In particular, the method may be provided that wherein thereby inducing an indel in the nucleic acid sequence in the gene of the hepatitis B infection.

To solve the above-described problems, the present application may provide a method for inactivating a conserved sequence in an HBV gene. Here, the HBV gene is one or more selected from the HBV genotypes consisting of HBV genotype A, HBV genotype B, HBV genotype C, HBV genotype D, HBV genotype E, HBV genotype F and HBV genotype G, and the conserved sequence is a sequence conserved in the HBV genotype sequences. Here, the conserved sequence is one or more selected from SEQ ID NOs. 1 to 45, and inactivation is cleavage of the conserved sequence.

The method may be provided that it comprises:
introducing into a subject a composition comprising:
   a) a guide nucleic acid, or a nucleic acid sequence encoding the same; and
   b) a Cas protein, or a nucleic acid sequence encoding the same;
the guide nucleic acid is a sequence having a complementarity or homology with the conserved sequence in part or in whole,
wherein the guide nucleic acid selected from SEQ ID NO.51, 52, 56, 57, 69, 70, 73, 74, 75, 76, 80, 85, 87 and 89.

### [Advantageous Effects]

According to the present application, the following effects are shown.

First, according to the present application, by targeting conserved regions of various HBV genotypes, a method for inhibiting HBV proliferation without being significantly limited by genotype can be provided.

Second, according to the present application, a composition for targeting conserved regions of various HBV genotypes can be provided. Here, therapeutic effects on the various HBV genotypes can be obtained using the same composition.

Third, according to the present application, conserved regions of various HBV genotypes can be provided.

### [Description of Drawings]

FIG. 1 is a schematic diagram for the design of CRISPR gRNAs which are capable of targeting HBV genotypes B, C and D.
FIG. 2 shows the virtual universal HBV genome of HBV genotype B manufactured based on an open database.
FIG. 3 shows the virtual universal HBV genome of HBV genotype C manufactured based on an open database.
FIG. 4 shows the virtual universal HBV genome of HBV genotype D manufactured based on an open database.
FIG. 5 shows the gRNA distribution of SpCas9(Streptococcus pyogenes Cas9) targeting virtual universal HBV genotypes B, C or D.
FIG. 6 shows the distribution of CjCas9(Campylobacter jejuni Cas9) targeting virtual universal HBV genotypes B, C or D.
FIG. 7 shows the gRNA distribution of SpCas9 targeting a commonly conserved region in sequences targeting the obtained open database-based conserved regions, sequences of Korean patient-derived HBV genotype C, and available HBV cell models.
FIG. 8 shows the gRNA distribution of CjCas9 targeting a commonly conserved region in sequences targeting the obtained open database-based conserved regions, sequences of Korean patient-derived HBV genotype C, and available HBV cell models.
FIG. 9 shows target regions of HBV gRNA of the present application.
FIGS. 10 to 13 and 18 show results of measuring HBsAg and HBeAg of the HBV D genotype in gRNA candidates (Cj#06, Cj#45, Cj#47, Cj#57) for CjCas9.
FIGS. 14 to 17 and 19 show results of measuring HBsAg and HBeAg of the HBV D genotype in gRNA candidates (Sp#17, Sp#20, Sp#89, Sp#90, Sp#154, Sp#159, Sp#193, Sp#194, Sp#196, Sp#197) for SpCas9.
FIGS. 20 and 21 show results of measuring HBsAg and HBeAg of Huh7 (HBV D genotype) and HepG2 (HBV D genotype) cell lines in gRNA candidates (Sp#17, Sp#90, Sp#193, Sp#197, Sp#17+90+193, Sp#17+90+197, Sp#17+193+197, Sp#90+193+197) for SpCas9.
FIG. 22 is a table that summarizes the results of FIGS. 20 and 21.

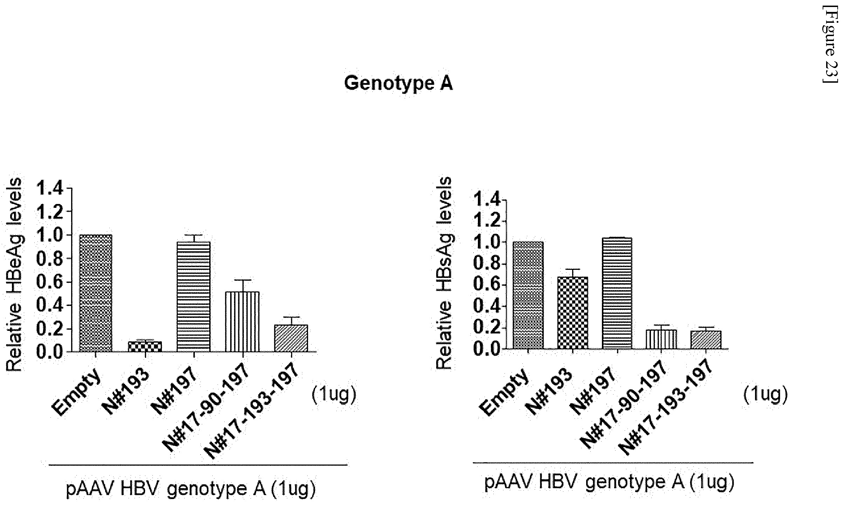

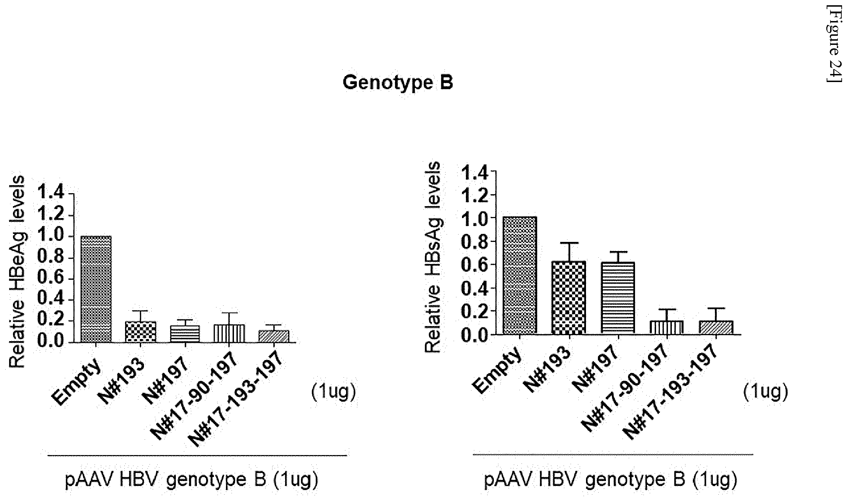

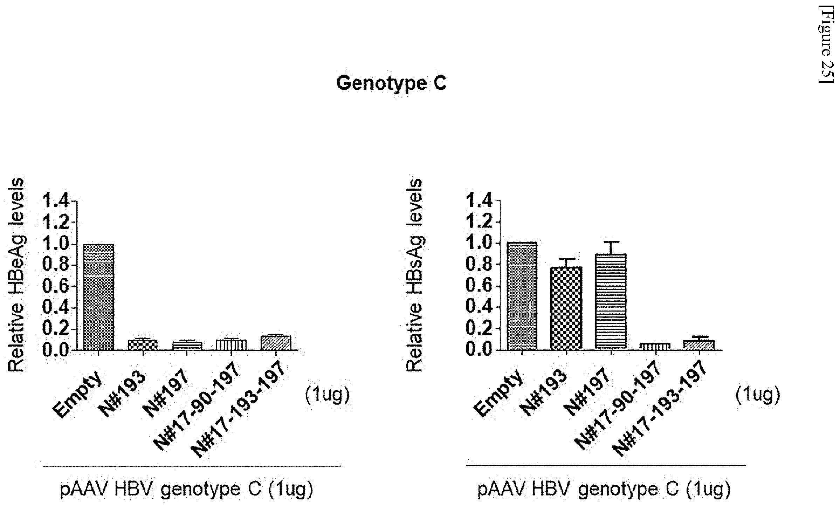

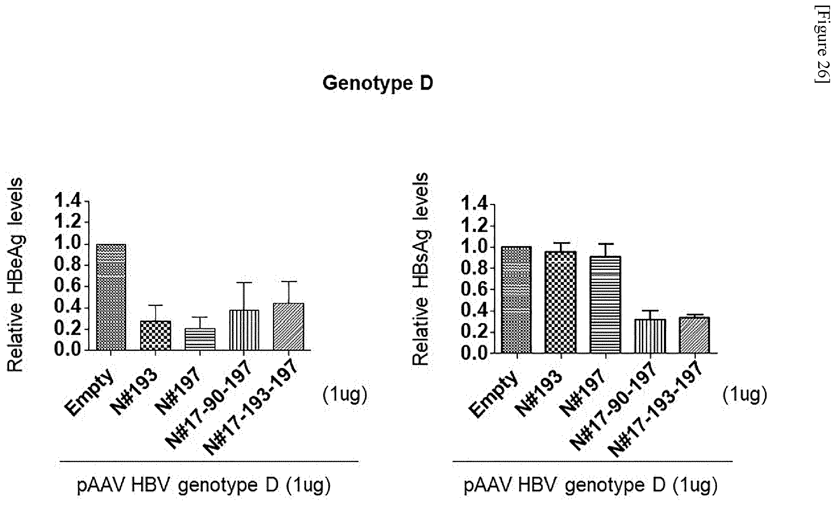

FIGS. 23 to 26 show results of measuring HBsAg and HBeAg of each of HBV genotype A, HBV genotype B, HBV genotype C and HBV genotype D in gRNA candidates (Sp#193, Sp#197, Sp#17+90+197, Sp#17+193+197) for SpCas9.
FIG. 27 is a vector map which is designed to express one SpgRNA, Cas9 and a fluorescent protein in one plasmid.
FIG. 28 is a vector map which is designed to express one CjgRNA, Cas9 and a fluorescent protein in one plasmid.
FIG. 29 is a vector map which is designed to express three SpgRNAs, Cas9 and a fluorescent protein in one plasmid.

### [Modes of the Invention]

Unless defined otherwise, all technical and scientific terms used in the specification have the same meanings as commonly understood by one of ordinary skill in the art to which the present invention belongs. Although methods and materials similar or equivalent to those described in the specification can be used in the practice or experiments of the present invention, suitable methods and materials are described below. All publications, patent applications, patents and other references mentioned in the specification are incorporated by reference in their entirety. In addition, the materials, methods and examples are merely illustrative and not intended to be limiting.

Hepatitis B is not only one of the most prevalent infectious diseases worldwide, but also a disease which is highly likely to develop into chronic hepatic diseases such as cirrhosis and liver cancer.

A virus causing hepatitis B, HBV, is a DNA virus belonging to the Hepadnaviridae family, and an incomplete double-stranded virus consisting of approximately 3,200 base pairs (3.2 kb).

HBV has plus-strand DNA with an incomplete complementary structure located inside minus-strand DNA with a complete circular structure.

In the HBV genome, there are four overlapping open reading frames (ORFs), and the frames have genes designating pre-S/S, C, P and X, respectively. The ORFs of the HBV genome have more than 50% overlapping parts. Particularly, HBV polymerase overlaps the entire pre-S/S, and overlaps other ORFs. Thus, mutations that occur in HBV replication may affect one or more ORFs, causing mutations to easily and particularly occur during HBV replication.

To date, it is known that there are a total of 7 types of various HBV genotypes A to G. Each subtype has an 8% or more difference in the entire base sequence of HBV.

The HBV genotype has a distinct distribution pattern depending on a region. Genotypes A and D are the major types in Western Europe and North America, and genotypes B and C are known as major types in Asia. GenoType E is only limited to Africa, and type F is common in Central America. Particularly, it has been reported that the virus proliferation of HBV genotypes B and C is maintained longer than that of other genotypes of HBVs (J Infect Dis 1997; 176:851-858).

Since there are various HBV genotypes, an effective method for inhibiting HBV proliferation without being limited by such genotypes is needed.

### • Difficulty in inhibiting proliferation of various HBV genotypes

As described above, HBV is not only the cause of diseases such as hepatitis and liver cancer, but also known to have a total of 7 genotypes.

When HBV enters the blood, it is generally located in liver cells, causing an immune response in our body to eliminate the HBV. Therefore, interferon α, which is the first developed hepatitis B therapeutic, was developed to promote the expression of a major histocompatibility complex I (MHC I) antigen in the HBV-infected liver cells and promote the disruption of the HBV-infected liver cells.

However, interferon α is effective only on some genotypes, not on all of the various HBV genotypes, and does not inactivate activated HBV DNA by integration into liver cells. Thus, there is a possibility of recurrence.

Recently, research to inhibit HBV proliferation by inactivating the integrated HBV DNA using a CRISPR/Cas system is being conducted (EP2014830911, World J Gastroenterol 2015 August 28; 21(32): 9554-9565).

Specifically, in EP2014830911, hundreds of guide nucleic acid candidates, which target a conserved region of HBV are disclosed. Among these, approximately 24 guide nucleic acid candidates selected for screening to confirm whether HBV proliferation is inhibited are disclosed. In addition, as a result of screening, among the approximately 24 guide nucleic acid candidates, only about three guide nucleic acids are reported to substantially inhibit HBV proliferation.

In World J Gastroenterol 2015 August 28; 21(32): 9554-9565, approximately 15 guide nucleic acid candidates targeting a conserved region of HBV are also disclosed. In this document, approximately 15 guide nucleic acids are also used alone or in combination for screening to confirm HBV inhibition, and as in EP2014830911, it is reported that the guide nucleic acid candidates have different effects. In addition, among the combination of specific guide nucleic acids, it has been reported that only some of them have various HBV genotypes inhibitory effects.

As such, according to several documents, among even the compositions targeting a conserved region of HBV, only some exhibit HBV proliferation inhibitory effects, and only a few guide nucleic acids can be applied to various HBV genotypes.

In other words, even in the case of targeting a conserved region of HBV, the HBV proliferation inhibitory effect appears only in specific sequences. Therefore, according to these known facts, it can be seen that only with effects of inhibiting the proliferation of some of various HBV genotypes, the effects of inhibiting the proliferation of other HBV genotypes cannot be easily expected.

Accordingly, in research to inhibit HBV proliferation by inactivating HBV DNA in the art, it is required to find a specific target sequence having a substantial proliferation inhibitory effect on various HBV genotypes.

In response to this need, the present application provides a method and composition for inhibiting the proliferation of various HBV genotypes. Particularly, the present application uses another specific target sequence having a substantial proliferation inhibitory effect on various HBV genotypes by extracting a conserved region of HBV from more various subjects than before and designing it to target these subj ects.

### • Technical features of the present application

The present application relates to a composition for targeting a specific conserved region of HBV and a method using the same, and particularly, to a composition and method for simultaneously inhibiting various HBV genotypes.

In the present application, a specific region on the targeted HBV genome is obtained by a method different from the related art, and such a specific region determined in the present application is clearly different from the region disclosed in the related art.

Specifically, a subject for extracting a specific conserved region of HBV is obtained by combining information of a conventional database, information derived from HBV-infected patients and information derived from HBV-injected cell lines.

By targeting a specific region on the HBV genome determined by the above-described method, a proliferation inhibitory effect on all of the various HBV genotypes may be exhibited.

Accordingly, in the present application, a treatment method effective on all genotypes using the same sequence (region), rather than a treatment method considering the sequence of each of various HBV genotypes, for example, HBV type A, HBV type B, HBV type C, HBV type D, HBV type E, HBV type F and HBV type G, may be applied. That is, HBV proliferation may be inhibited with one type of composition without specific limitation to various HBV genotypes.

Therefore, the present application has the following technical features.

First, the method for inhibiting HBV proliferation of the present application inactivates a specific sequence conserved in an HBV gene. Here, the specific sequence conserved is a sequence conserved in a region in which mutations do not easily occur between sequences of various HBV genotypes. Thus, the method for the present application may be applied without limitation to various HBV genotypes. In the present application, at least two HBV genotypes selected from HBV genotype A, HBV genotype B, HBV genotype C, HBV genotype D, HBV genotype E, HBV genotype F and HBV genotype G may be simultaneously inhibited.

Second, according to the composition and method for inhibiting HBV proliferation by targeting a specific region of the present application, a therapeutic effect may be exhibited using one type of specific region of various HBV genotypes. That is, using the same composition and method, hepatitis B caused by HBV genotype A, HBV genotype B, HBV genotype C, HBV genotype D, HBV genotype E, HBV genotype F or HBV genotype G may be treated.

Hereinafter, a method for inhibiting the proliferation of various HBV genotypes having the technical features of the present application will be described in further detail.

### Proliferation inhibitory method using common target for hepatitis B caused by various HBV genotypes

One aspect of the present application relates to a method for inhibiting the proliferation of various HBV genotypes using a common target region.

Specifically, the method for inhibiting HBV proliferation of the present application is to inhibit HBV proliferation using a technique of targeting a specific region commonly conserved in various HBV genotypes.

The HBV genotype may be selected from HBV genotype A, HBV genotype B, HBV genotype C, HBV genotype D, HBV genotype E, HBV genotype F and HBV genotype G, but the present invention is not limited thereto.

The method for inhibiting HBV proliferation of the present application may simultaneously inhibit the proliferation of one or more HBV genotypes selected from HBV genotype A, HBV genotype B, HBV genotype C, HBV genotype D, HBV genotype E, HBV genotype F and HBV genotype G.

The term "conserved region" used herein should be interpreted as follows. The conserved region refers to a part in which mutations do not easily occur between sequences of at least two genotypes selected from HBV genotypes such as HBV genotype A, HBV genotype B, HBV genotype C, HBV genotype D, HBV genotype E, HBV genotype F and HBV genotype G. Particularly, the conserved region refers to a conserved region between base sequences of an HBV genotype selected from targets for obtaining base sequences of HBV genotypes by considering all of HBV-infected patient-derived cells and HBV-injected cell models as well as a database. In the specification, the nucleic acid sequence in the conserved region is referred to as a "conserved sequence."

The conserved sequence of the present application may be obtained using the following method.

According to one embodiment, a method for finding a conserved sequence of the various HBV genotypes may include:
aligning base sequences of HBV genotype B, HBV genotype C and HBV genotype D obtained from a database; and
finding conserved sequences by comparing the aligned HBV genotype B, HBV genotype C and HBV genotype D sequences.

The conserved sequence obtained by the above-described method may be one or more selected from SEQ ID NOs. 1 to 45 of Table 1 below.

**[Table 1]**

| **Name** | **sequence (5' → 3')** | **PAM** |
|---|---|---|
| Sp20-viHBV-B-#10 | GTAACACGAGCAGGGGTCCT **(SEQ ID NO. 1)** | AGG |
| Sp20-viHBV-B-#11 | CCCCGCCTGTAACACGAGCA **(SEQ ID NO. 2)** | GGG |
| Sp20-viHBV-B-#12 | ACCCCGCCTGTAACACGAGC **(SEQ ID NO. 3)** | AGG |
| Sp20-viHBV-B-#13 | AGGACCCCTGCTCGTGTTAC **(SEQ ID NO. 4)** | AGG |
| Sp20-viHBV-B-#14 | ACCCCTGCTCGTGTTACAGG **(SEQ ID NO. 5)** | CGG |
| Sp20-viHBV-B-#17 | CACCACGAGTCTAGACTCTG **(SEQ ID NO. 6)** | TGG |
| Sp20-viHBV-B-#20 | GGACTTCTCTCAATTTTCTA **(SEQ ID NO. 7)** | GGG |
| Sp20-viHBV-B-#52 | CCTACGAACCACTGAACAAA **(SEQ ID NO. 8)** | TGG |
| Sp20-viHBV-B-#53 | CCATTTGTTCAGTGGTTCGT **(SEQ ID NO. 9)** | AGG |
| Sp20-viHBV-B-#54 | CATTTGTTCAGTGGTTCGTA **(SEQ ID NO. 10)** | GGG |
| Sp20-viHBV-B-#89 | GGGTTGCGTCAGCAAACACT **(SEQ ID NO. 11)** | TGG |
| Sp20-viHBV-B-#90 | TTTGCTGACGCAACCCCCAC **(SEQ ID NO. 12)** | TGG |
| Sp20-viHBV-B-#101 | TCCGCAGTATGGATCGGCAG **(SEQ ID NO. 13)** | AGG |
| Sp20-viHBV-B-#102 | AGGAGTTCCGCAGTATGGAT **(SEQ ID NO. 14)** | CGG |
| Sp20-viHBV-B-#103 | TCCTCTGCCGATCCATACTG **(SEQ ID NO. 15)** | CGG |
| Sp20-viHBV-B-#113 | CGTCCCGCGCAGGATCCAGT **(SEQ ID NO. 16)** | TGG |
| Sp20-viHBV-B-#117 | CCGCGGGATTCAGCGCCGAC **(SEQ ID NO. 17)** | GGG |
| Sp20-viHBV-B-#118 | TCCGCGGGATTCAGCGCCGA **(SEQ ID NO. 18)** | CGG |
| Sp20-viHBV-B-#119 | CCCGTCGGCGCTGAATCCCG **(SEQ ID NO. 19)** | CGG |
| Sp20-viHBV-B-#138 | GTAAAGAGAGGTGCGCCCCG **(SEQ ID NO. 20)** | TGG |
| Sp20-viHBV-B-#140 | GGGGCGCACCTCTCTTTACG **(SEQ ID NO. 21)** | CGG |
| Sp20-viHBV-B-#142 | GAAGCGAAGTGCACACGGTC **(SEQ ID NO. 22)** | CGG |
| Sp20-viHBV-B-#143 | GGTCTCCATGCGACGTGCAG **(SEQ ID NO. 23)** | AGG |
| Sp20-viHBV-B-#154 | AATGTCAACGACCGACCTTG **(SEQ ID NO. 24)** | AGG |
| Sp20-viHBV-B-#159 | AGGAGGCTGTAGGCATAAAT **(SEQ ID NO. 25)** | TGG |
| Sp20-viHBV-B-#186 | CGGAAGTGTTGATAAGATAG **(SEQ ID NO. 26)** | GGG |
| Sp20-viHBV-B-#187 | CCGGAAGTGTTGATAAGATA **(SEQ ID NO. 27)** | GGG |
| Sp20-viHBV-B-#193 | GCGAGGGAGTTCTTCTTCTA **(SEQ ID NO. 28)** | GGG |
| Sp20-viHBV-B-#194 | GACCTTCGTCTGCGAGGCGA **(SEQ ID NO. 29)** | GGG |
| Sp20-viHBV-B-#196 | GATTGAGACCTTCGTCTGCG **(SEQ ID NO. 30)** | AGG |
| Sp20-viHBV-B-#197 | CTCCCTCGCCTCGCAGACGA **(SEQ ID NO. 31)** | AGG |
| Sp20-viHBV-B-#198 | GATTGAGATCTTCTGCGACG **(SEQ ID NO. 32)** | CGG |
| Sp20-viHBV-B-#199 | GTCGCAGAAGATCTCAATCT **(SEQ ID NO. 33)** | CGG |
| Sp20-viHBV-B-#200 | TCGCAGAAGATCTCAATCTC **(SEQ ID NO. 34)** | GGG |
| Cj22-viHBV-B-#06 | TGTCAACAAGAAAAACCCCGCC **(SEQ ID NO. 35)** | TGTAACAC |
| Cj22-viHBV-B-#20 | AAGCCCTACGAACCACTGAACA **(SEQ ID NO. 36)** | AATGGCAC |
| Cj22-viHBV-B-#23 | TTACCAATTTTCTTTTGTCTTT **(SEQ ID NO. 37)** | GGGTATAC |
| Cj22-viHBV-B-#40 | ACGTCCCGCGCAGGATCCAGTT **(SEQ ID NO. 38)** | GGCAGCAC |
| Cj22-viHBV-B-#44 | GTGCACACGGTCCGGCAGATGA **(SEQ ID NO.** | GAAGGCAC |
| | **39)** | |
| Cj22-viHBV-B-#45 | GTGCCTTCTCATCTGCCGGACC **(SEQ ID NO. 40)** | GTGTGCAC |
| Cj22-viHBV-B-#46 | CGACGTGCAGAGGTGAAGCGAA **(SEQ ID NO. 41)** | GTGCACAC |
| Cj22-viHBV-B-#47 | TGCGACGTGCAGAGGTGAAGCG **(SEQ ID NO. 42)** | AAGTGCAC |
| Cj22-viHBV-B-#48 | GACCGTGTGCACTTCGCTTCAC **(SEQ ID NO. 43)** | CTCTGCAC |
| Cj22-viHBV-B-#57 | ATGTCCATGCCCCAAAGCCACC **(SEQ ID NO. 44)** | CAAGGCAC |
| Cj22-viHBV-B-#67 | GACCACCAAATGCCCCTATCTT **(SEQ ID NO. 45)** | ATCAACAC |

As another example, the method for finding the conserved sequence of the various HBV genotypes may include:
obtaining base sequences of the various HBV genotypes from the following base sequences, such as
   i) base sequences derived from databases of HBV genotype B, HBV genotype C and HBV genotype D, respectively,
   ii) a base sequence of an HBV genotype D cell model, and
   iii) a base sequence derived from an HBV type C-infected patient;
aligning the base sequences of the i), ii), iii); and
finding conserved sequences in the aligned sequences.

The conserved sequence of the present application obtained by the above-described method may be any one or more selected from SEQ ID NOs. 6, 7, 11, 12, 24, 25, 28, 29, 30, 31, 35, 40, 42 and 44 in Table 1.

The most preferable method for finding a conserved sequence of various HBV genotypes according to the present application is, as described above, to obtain a conserved sequence by expanding subjects acquiring base sequences of various HBV genotypes.

According to this method, the common conserved region of the present application, which may exhibit an inhibitory effect on various HBV genotypes at the same time, may be determined. The determined conserved sequence may be sequences selected from SEQ ID NOs. 1 to 45, and more preferably, sequences selected from SEQ ID NOs. 6, 7, 11, 12, 24, 25, 28, 29, 30, 31, 35, 40, 42 and 44; and a sequence having 70% or more, 75% or more, 80% or more, 85% or more, 90% or more, 95% or more, or 100% homology with respect to the sequences.

Likewise, the conserved sequence of HBV targeted by the method described above, preferably, base sequence data for various HBV genotypes may be obtained from a combination of various subjects derived from databases, patients, and cell models.

The specific conserved sequence determined as described above may be a target capable of inhibiting HBV proliferation without limitation to an HBV genotype unlike the conventional case.

A composition of the present application which will be described in further detail below is designed to target the conserved region of the various HBV genotypes.

One embodiment of the present application relates to a composition for inhibiting the proliferation of various HBV genotypes.

The composition targets conserved regions of various HBV genotypes.

The term "targets" refers to a mechanism showing an HBV proliferation inhibitory effect or killing effect by inducing the modification or inactivation of function of a sequence of the conserved region of HBV.

The composition of the present application for inhibiting the proliferation of various HBV genotypes is not limited as long as it has a function of targeting the common specific region determined above, that is, the conserved region (sequence). For example, the composition of the present application may include both of a composition capable of inducing a mutation of a conserved region on HBV genomic DNA, and a composition capable of inhibiting the translation of a conserved region on HBV mRNA.

In one embodiment, the composition of the present application includes a CRISPR system.

Specifically, a guide nucleic acid which target the conserved region of the present application and a Cas protein may be included.

The CRISPR system is a system that is capable of introducing an artificial mutation by targeting a target sequence around a proto-spacer-adjacent motif (PAM) sequence on genomic DNA. Specifically, the guide nucleic acid and the Cas protein may bind to (or interact with) each other, thereby forming a guide nucleic acid-Cas protein complex, and a mutant indel may be induced on the genomic DNA by cleaving a desired DNA sequence. The composition of the present application may inhibit the proliferation or function of HBV by introducing an indel into the above-described conserved region.

For more detailed description on the guide nucleic acid, the Cas protein and the guide nucleic acid-Cas protein complex, Korean Patent No. 10-2017-0126636 may be referred to.

In the present application, the Cas protein may include one or more proteins selected from a *Streptococcus pyogenes* (Sp)-derived Cas9 protein, and a *Campylobacter jejuni* (Cj)-derived Cas9 protein, or one or more selected from nucleic acid sequences encoding the same. The Cas9 protein serves to cleave a DNA sequence at a PAM sequence-adjacent position by recognizing a PAM(proto-spacer-adjacent Motif) sequence on the genomic DNA sequence of a subject and interacting with a guide nucleic acid.

In the present application, the guide nucleic acid may complementarily bind to the conserved sequence of the present application, as a target sequence, of the HBV genomic DNA sequence.

The guide nucleic acid includes a site complementarily binding to the target sequence (referred to as a guide region) and a site involved in forming a complex with a Cas protein (referred to as a complex-forming region).

Here, the guide region may be a sequence having homology or complementarity with a sequence selected from SEQ ID NOs. 1 to 45, more preferably, SEQ ID NOs. 6, 7, 11, 12, 24, 25, 28, 29, 30, 31, 35, 40, 42 and 44. Although the guide region includes 0 to 5 mismatches while binding to the target sequence, it is not a big issue if the complex function is not affected.

The sequence targeting the conserved sequence of HBV may be any one selected from SEQ ID NOs.46 to 90 in Table 2.

In one example, the guide nucleic acid may be any one interacting with a SpCas9 protein and selected from SEQ ID NOs.46 to 79.

In another example, the guide nucleic acid may include any one interacting with a CjCas9 protein and selected from SEQ ID NOs.80 to 90.

Here, the complex-forming region may be determined by the type of Cas9 protein-derived microorganism. For example, in the case of the guide nucleic acid interacting with the SpCas9 protein, the complex-forming region may include 5'-GUUUUAGUCCCUGAAAAGGGACUAAAAUAAAGAGUUUGCGGGACUCU GCGGGGUUACAAUCCCCUAAAACCGCUUUU-3', and in the case of the guide nucleic acid interacting with the CjCas9 protein, include 5'-GUUUUAGAGCUAGAAAUAGCAAGUUAAAAUAAGGCUAGUCCGUUAUC AACUUGAAAAAGUGGCACCGAGUCGGUGC-3'.

**[Table 2]**

| **NO.** | **Name** | **sequence (5' → 3')** |
|---|---|---|
| **SEQ ID NO. 46** | Sp20-viHBV-B-#10G | GUAACACGAGCAGGGGUCCU |
| **SEQ ID NO. 47** | Sp20-viHBV-B-#11G | CCCCGCCUGUAACACGAGCA |
| **SEQ ID NO. 48** | Sp20-viHBV-B-#12G | ACCCCGCCUGUAACACGAGC |
| **SEQ ID NO. 49** | Sp20-viHBV-B-#13G | AGGACCCCUGCUCGUGUUAC |
| **SEQ ID NO. 50** | Sp20-viHBV-B-#14G | ACCCCUGCUCGUGUUACAGG |
| **SEQ ID NO. 51** | Sp20-viHBV-B-#17G | CACCACGAGUCUAGACUCUG |
| **SEQ ID NO. 52** | Sp20-viHBV-B-#20G | GGACUUCUCUCAAUUUUCUA |
| **SEQ ID NO. 53** | Sp20-viHBV-B-#52G | CCUACGAACCACUGAACAAA |
| **SEQ ID NO. 54** | Sp20-viHBV-B-#53G | CCAUUUGUUCAGUGGUUCGU |
| **SEQ ID NO. 55** | Sp20-viHBV-B-#54G | CAUUUGUUCAGUGGUUCGUA |
| **SEQ ID NO. 56** | Sp20-viHBV-B-#89G | GGGUUGCGUCAGCAAACACU |
| **SEQ ID NO. 57** | Sp20-viHBV-B-#90G | UUUGCUGACGCAACCCCCAC |
| **SEQ ID NO. 58** | Sp20-viHBV-B-#101G | UCCGCAGUAUGGAUCGGCAG |
| **SEQ ID NO. 59** | Sp20-viHBV-B-#102G | AGGAGUUCCGCAGUAUGGAU |
| **SEQ ID NO. 60** | Sp20-viHBV-B-#103G | UCCUCUGCCGAUCCAUACUG |
| **SEQ ID NO. 61** | Sp20-viHBV-B-#113G | CGUCCCGCGCAGGAUCCAGU |
| **SEQ ID NO. 62** | Sp20-viHBV-B-#117G | CCGCGGGAUUCAGCGCCGAC |
| **SEQ ID NO. 63** | Sp20-viHBV-B-#118G | UCCGCGGGAUUCAGCGCCGA |
| **SEQ ID NO. 64** | Sp20-viHBV-B-#119G | CCCGUCGGCGCUGAAUCCCG |
| **SEQ ID NO. 65** | Sp20-viHBV-B-#138G | GUAAAGAGAGGUGCGCCCCG |
| **SEQ ID NO. 66** | Sp20-viHBV-B-#140G | GGGGCGCACCUCUCUUUACG |
| **SEQ ID NO. 67** | Sp20-viHBV-B-#142G | GAAGCGAAGUGCACACGGUC |
| **SEQ ID NO. 68** | Sp20-viHBV-B-#143G | GGUCUCCAUGCGACGUGCAG |
| **SEQ ID NO. 69** | Sp20-viHBV-B-#154G | AAUGUCAACGACCGACCUUG |
| **SEQ ID NO. 70** | Sp20-viHBV-B-#159G | AGGAGGCUGUAGGCAUAAAU |
| **SEQ ID NO. 71** | Sp20-viHBV-B-#186G | CGGAAGUGUUGAUAAGAUAG |
| **SEQ ID NO. 72** | Sp20-viHBV-B-#187G | CCGGAAGUGUUGAUAAGAUA |
| **SEQ ID NO. 73** | Sp20-viHBV-B-#193G | GCGAGGGAGUUCUUCUUCUA |
| **SEQ ID NO. 74** | Sp20-viHBV-B-#194G | GACCUUCGUCUGCGAGGCGA |
| **SEQ ID NO. 75** | Sp20-viHBV-B-#196G | GAUUGAGACCUUCGUCUGCG |
| **SEQ ID NO. 76** | Sp20-viHBV-B-#197G | CUCCCUCGCCUCGCAGACGA |
| **SEQ ID NO. 77** | Sp20-viHBV-B-#198G | GAUUGAGAUCUUCUGCGACG |
| **SEQ ID NO. 78** | Sp20-viHBV-B-#199G | GUCGCAGAAGAUCUCAAUCU |
| **SEQ ID NO. 79** | Sp20-viHBV-B-#200G | UCGCAGAAGAUCUCAAUCUC |
| **SEQ ID NO. 80** | Cj22-viHBV-B-#06G | UGUCAACAAGAAAAACCCCGCC |
| **SEQ ID NO. 81** | Cj22-viHBV-B-#20G | AAGCCCUACGAACCACUGAACA |
| **SEQ ID NO. 82** | Cj22-viHBV-B-#23G | UUACCAAUUUUCUUUUGUCUUU |
| **SEQ ID NO. 83** | Cj22-viHBV-B-#40G | ACGUCCCGCGCAGGAUCCAGUU |
| **SEQ ID NO. 84** | Cj22-viHBV-B-#44G | GUGCACACGGUCCGGCAGAUGA |
| **SEQ ID NO. 85** | Cj22-viHBV-B-#45G | GUGCCUUCUCAUCUGCCGGACC |
| **SEQ ID NO. 86** | Cj22-viHBV-B-#46G | CGACGUGCAGAGGUGAAGCGAA |
| **SEQ ID NO. 87** | Cj22-viHBV-B-#47G | UGCGACGUGCAGAGGUGAAGCG |
| **SEQ ID NO. 88** | Cj22-viHBV-B-#48G | GACCGUGUGCACUUCGCUUCAC |
| **SEQ ID NO. 89** | Cj22-viHBV-B-#57G | AUGUCCAUGCCCCAAAGCCACC |
| **SEQ ID NO. 90** | Cj22-viHBV-B-#67G | GACCACCAAAUGCCCCUAUCUU |

A guide region sequence (SEQ ID NOs. 46 to 90) of the guide nucleic acid of the present application is any one of the conserved sequences listed in Table 1 above, and designed by considering the PAM sequence located adjacent to the conserved sequence.

As the PAM sequence, when the spCas9 protein is used, NGG (N is A, T, C or G) is considered, and when the cjCas9 protein is used, NNNNRYAC (N is each independently A, T, C or G, R is A or G, and Y is C or T) is considered.

The composition may include one or more guide nucleic acids.

In one example, the guide nucleic acid of the present application may be used by selecting one or more from SEQ ID NOs. 46 to 90 of Table 2. Preferably, the guide nucleic acid of the present application is used by selecting a combination of two or more of the above sequences.

In one embodiment, the guide nucleic acid of the present application may be used by selecting one or a combination of two or more of SEQ ID NOs. 51, 52, 56, 57, 69, 70, 73, 74, 75, 76, 80, 85, 87 and 89. In one embodiment, the guide nucleic acid of the present application may use a combination of SEQ ID NOs. 51, 57 and 73 or a combination of SEQ ID NOs. 51, 73 and 76.

The composition of the present application may include, for example,
a guide nucleic acid including one or more selected from SEQ ID NOs. 51, 52, 56, 57, 69, 70, 73, 74, 75 and 76 or a nucleic acid sequence encoding the same; and a SpCas protein or a nucleic acid sequence encoding the same.

Alternatively, the composition of the present application may include a guide nucleic acid including one or more selected from SEQ ID NOs. 80, 85, 87 and 89 or a nucleic acid sequence encoding the same; and a CjCas protein or a nucleic acid sequence encoding the same.

The composition of the present application may target
all of two or more conserved regions of HBV genotypes selected from HBV genotype A, HBV genotype B, HBV genotype C, HBV genotype D, HBV genotype E, HBV genotype F and HBV genotype G.

In one example,
all of the conserved regions of the HBV genotype B, HBV genotype C and HBV genotype D may be targeted.

A form of the composition of the present application may be a vector or ribonucleoprotein (RNP) form. Therefore, the composition includes
a) a guide nucleic acid or a nucleic acid sequence encoding the guide nucleic acid; and
b) a Cas protein or a nucleic acid sequence encoding theCas protein.

Here, a composition in the form of a vector may be a vector simultaneously or separately including a guide nucleic acid and/or a nucleic acid sequence encoding a Cas protein.

For example, the vector may include both of a guide nucleic acid and a nucleic acid sequence encoding a Cas protein, and as another example, the vector may individually include a guide nucleic acid and a nucleic acid sequence encoding a Cas protein.

Here, one or more guide nucleic acids and/or Cas proteins may be used. In one embodiment, two or three guide nucleic acids may be combined.

The vector may be a viral vector or a plasmid.

The viral vector may be one or more selected from the group consisting of a retrovirus, a lentivirus, an adenovirus, an adeno-associated virus (AAV), a vaccinia virus, a pox virus and herpes simplex virus.

In one embodiment, in the composition,
the guide nucleic acid in a) may be any one selected from SEQ ID NOs. 51, 52, 56, 57, 69, 70, 73, 74, 75 and 76, and
the Cas protein in b) may include a *Streptococcus pyogenes-derived* Cas9 protein. Here, in one embodiment, the guide nucleic acid may use a combination of SEQ ID NOs. 51, 57 and 73 or a combination of SEQ ID NOs. 51, 73 and 76.

In another embodiment, the composition may include
the guide nucleic acid in a) may be any one selected from SEQ ID NOs. 80, 85, 87 and 89, and
the Cas protein in b) may include a *Campylobacter jejuni-*derived Cas9 protein.
Meanwhile, the composition may be prepared in the form of an RNP

This means a complex form in which the guide nucleic acid RNA sequence of the present application binds to (or interacts with) a Cas protein.

The composition of the present application may inhibit the proliferation of the virus regardless of the genotype of a virus by targeting specific conserved regions of various HBV genotypes.

The complex of the guide nucleic acid and the Cas protein of the composition targets a conserved region in HBV genomic DNA to cleave the double strands in the conserved region. As a result, due to an indel in the conserved region, that is, an artificial mutation, the virus is inactivated, thereby inhibiting HBV proliferation.

As a result of the inactivation, both of knockdown and knockout are included.

The "inactivation" refers to the prevention of normal expression of a target gene or a nucleic acid encoding the same in cells, or disabling or inhibition of the function thereof. The inactivation may be performed by inducing a state in which a target gene or nucleic acid is not transcribed and/or translated.

The "knockout" includes particularly a method for deleting genetic information, for example, cleaving a fragment of genetic DNA. Through the knockout, it is possible to inhibit the transcription and/or translation of a disease-causing gene or a gene with an abnormal function.

The "knockdown" particularly refers to blocking of the translation of a gene into a protein, and may include blocking of the generation of a protein from mRNA using a specific material such as iRNA or miRNA.

The composition of the present application, particularly, may generate an indel by NHEJ on an HBV genome by introducing DNA cleavage in a target sequence. Accordingly, an effect of inhibiting the proliferation or function of HBV may be exhibited.

In this application, when the conserved region of HBV is targeted, HBV proliferation may be inhibited, and the generation or expression of an HBV antigen may be inhibited.

The composition of the present application may inhibit simultaneous proliferation of two or more HBV genotypes selected from HBV genotype A, HBV genotype B, HBV genotype C, HBV genotype D, HBV genotype E, HBV genotype F and HBV genotype G. In addition, the generation or expression of one or more HBV antigens selected from HBsAg, HBeAg and HBcAg may be inhibited.

Another aspect of the present application relates to a method for inhibiting HBV proliferation using the composition.

The method for inhibiting HBV proliferation of the present invention may be performed by introducing or administering the composition of the present application to a subject, and here, the composition may include:
a) a guide nucleic acid or a nucleic acid sequence encoding the guide nucleic acid; and
b) a Cas protein or a nucleic acid sequence encoding theCas protein.

Here, the guide nucleic acid may include any one or more selected from SEQ ID NOs. 51, 52, 56, 57, 69, 70, 73, 74, 75, 76, 80, 85, 87 and 89. Here, an indel is formed in a nucleic acid sequence in the HBV gene by the composition. According to this method, the proliferation of two or more HBV genotypes selected from HBV genotype A, HBV genotype B, HBV genotype C, HBV genotype D, HBV genotype E, HBV genotype F and HBV genotype G may be inhibited at the same time.

The method for the present application may inhibit proliferation of several types of HBVs of the various HBV genotypes with the same composition of the present application.

Meanwhile, the present application relates to a method for inactivating a conserved sequence in an HBV gene.

The method is characterized by targeting a specific conserved sequence regardless of an HBV genotype. The conserved sequence is a sequence conserved between sequences of the HBV genotype to inactivate, for example, one or more selected from the conserved sequences of SEQ ID NOs. 1 to 45, and preferably, SEQ ID NOs. 6, 7, 11, 12, 24, 25, 28, 29, 30, 31, 35, 40, 42 and 44. Here, the inactivation includes cleavage of the conserved sequence.

This method may inactivate one or more types of viruses selected from HBV genotype A, HBV genotype B, HBV genotype C, HBV genotype D, HBV genotype E, HBV genotype F and HBV genotype G regardless of a genotype.

In one embodiment of the present application, to perform this method,
a) a guide nucleic acid or a nucleic acid sequence encoding the guide nucleic acid; and
b) a Cas protein or a nucleic acid sequence encoding the cas protein may be introduced or administered to a subject. In one embodiment, the guide nucleic acid may include one or more selected from SEQ ID NOs. 51, 52, 56, 57, 69, 70, 73, 74, 75, 76, 80, 85, 87 and 89. In one embodiment, the guide nucleic acid may use a combination of SEQ ID NOs. 51, 57 and 73 or a combination of SEQ ID NOs. 51, 73 and 76.

The method of the present application inactivates a conserved sequence in the HBV gene. Here, since this method uses a conserved sequence in which mutations between sequences of various HBV genotypes do not occur easily, HBV proliferation may be inhibited by applying the method of the present application without limitation to various HBV genotypes.

The method of the present invention has an effect of treating hepatitis B.

The method of the present invention may be performed by introducing or administering the composition of the present application to a subject in need of administration thereof.

The subject may be an HBV-infected mammal, for example, a human, a monkey, a mouse or a rat, but the present invention is not limited thereto.

Here, the method of the present application may be performed when the subject is infected by any of HBV genotype A, HBV genotype B, HBV genotype C, HBV genotype D, HBV genotype E, HBV genotype F and HBV genotype G. Although unusual, the subject may be co-infected by two or more thereof.

That is, since the present application has an effect of simultaneously inhibiting various HBV genotypes, when the composition and method of the present application are used, HBV proliferation may be effectively inhibited and hepatitis B may be treated regardless of a specific type of the infected HBV.

The administration may be performed by any convenient method such as injection, transfection, implantation or transplantation. The administration route may be selected from subretinal, subcutaneous, intradermal, intraocular, intravitreal, intratumoral, intranodal, intramedullary, intramuscular, intravenous, intralymphatic, and intraperitoneal administrations.

During administration, a dose (a pharmaceutically effective amount to obtain a desired effect) of the composition may be selected from all integers within the numerical ranges including approximately 10⁴ to 10⁹ cells, e.g., 10⁵ to 10⁶ cells/kg (body weight of a subject), but the present invention is not limited thereto. The dose of the present application may be suitably prescribed by considering the age, health and body weight of a subject, the type of concurrent treatment, if any, treatment frequency, or the characteristics of a desired effect.

### [Examples]

Hereinafter, the present invention will be described in further detail with reference to examples.

The examples are merely provided to more specifically explain the present invention, and it will be obvious to those of ordinary skill in the art that the scope of the present invention is not limited to the examples according to the gist of the present invention.

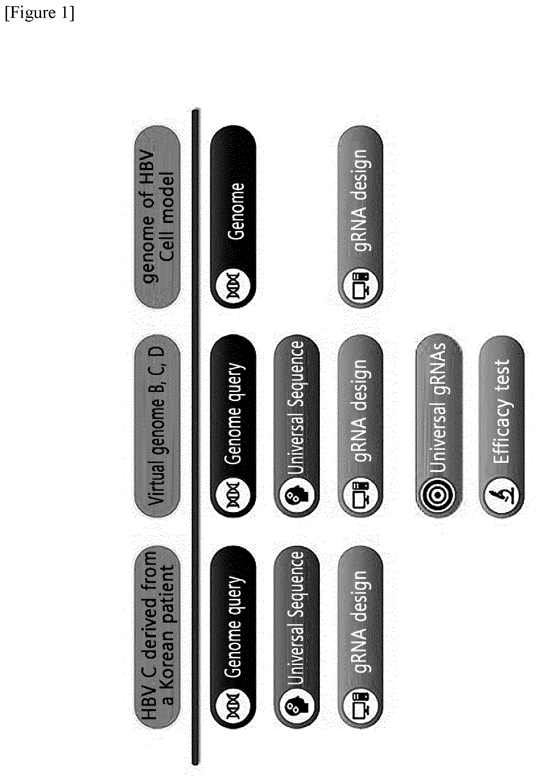

The diagram for designing a composition for targeting various HBV genotypes according to the present application is shown in FIG. 1. FIG. 1 is a schematic diagram for designing CRISPR gRNAs which are able to target HBV genotypes B, C and D.

As shown in FIG. 1, a genetic scissors which is able to multi-target all of genomes of Korean patient-derived HBV genotype C, open database-derived HBV genotypes B, C and D, and an HBV cell model.

The schematic diagram of FIG. 1 will be described in detail below.

### Example 1. Genome query & alignment for HBV genotypes B, C and D

For the genome query for HBV types B, C and D, 1638, 2136 and 923 full sequences of HBV types B, C and D, respectively, were collected from an HBV genome data base (HBVdb, https://hbvdb.lyon.inserm.fr/HBVdb/HBVdbIndex). A virtual HBV genome (hereinafter, vHBV) sequence was made through sequence alignment of the collected sequences.

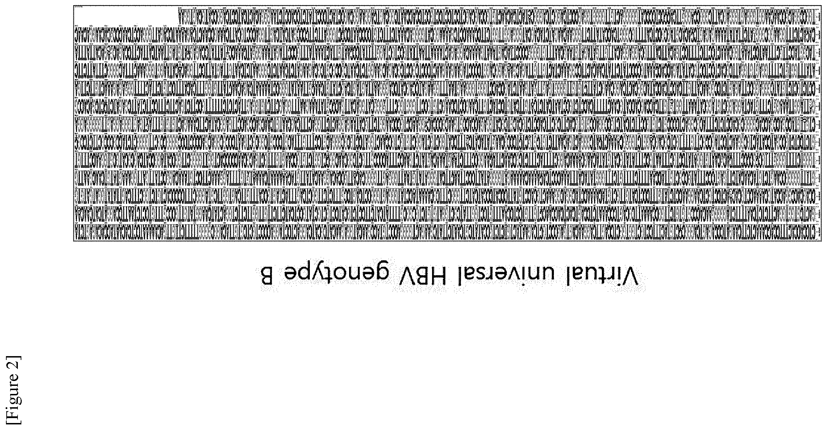

The virtual HBV genome sequence type B, C or D was manufactured based on a region with 80% sequence homology among aligned sequences of each of the full sequences of HBV type B, C or D (FIGS. 2 to 4).

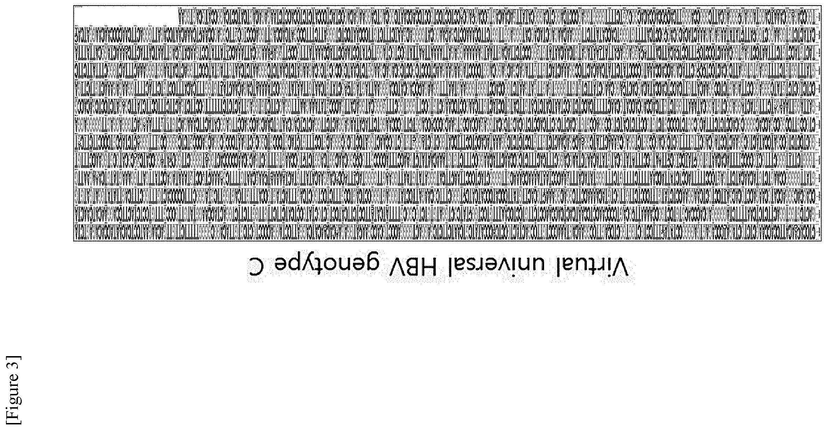

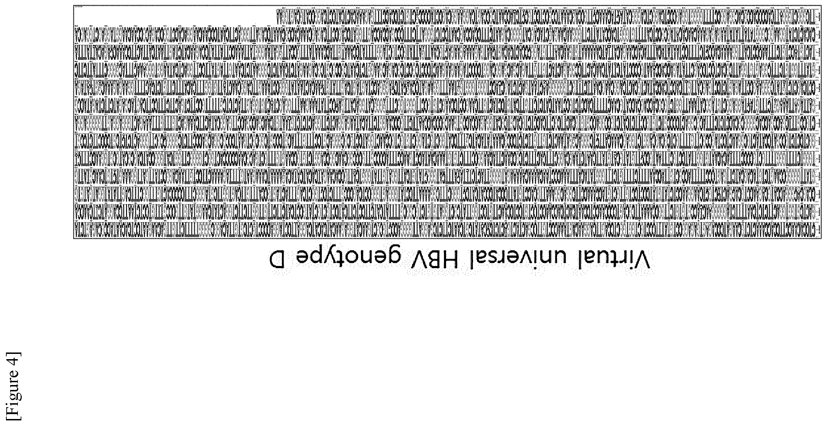

SEQ ID NO. 91 corresponds to the sequence of FIG. 2, SEQ ID NO. 92 corresponds to the sequence of FIG. 3, and SEQ ID NO. 93 corresponds to the sequence of FIG. 4. In the sequences of SEQ ID NOs. 91 to 93, the region indicated by capital letters has 80% or more homology with a sequence analyzed in the present application, and the region indicated by small letters has 80% or less homology.

**[Table 3]**

| |
|---|
| **virtual HBV B genotype sequence (****FIG. 2****) SEQ ID NO. 91** |
| CTCCACCACTTTCCACCAAACTCTTCAAGATCCCAGAGTCAGGGCcCTGTACT TTCCTGCTGGTGGCTCCAGTTCAGGAACAGTGAGCCCTGCTCAGAATACTGtCTCTGCC ATATCGTCAATCTTATCGAAGACTGGGGACCCTGTACCGAACATGGAGAACATCGCAT CAGGACTCCTAGGACCCCTGCTCGTGTTACAGGCGGGGTTTTTCTTGTTGACAAAAATC CTCACAATACCACAGAGTCTAGACTCGTGGTGGACTTCTCTCAATTTTCTAGGGGGAA CACCCGTGTGTCTTGGCCAAAATTCGCAGTCCCAAATCTCCAGTCACTCACCAACcTGT TGTCCTCCAATTTGTCCTGGTTATCGCTGGATGTGTCTGCGGCGTTTTATCATCTTCCTC TGCATCCTGCTGCTATGCCTCATCTTCTTGTTGGTTCTTCTGGACTATCAAGGTATGTTG CCCGTTTGTCCTCTAATTCCAGGATCATCAACaACCAGCACCGGACCATGCAAAACCTG CACaACTCCTGCTCAAGGAACCTCTATGTTTCCCTCATGTTGCTGTACAAAACCTACGG |
| ACGGAAACTGCACCTGTATTCCCATCCCATCATCTTGGGCTTTCGCAAAATACCTATGG GAGTGGGCCTCAGTCCGTTTCTCTTGGCTCAGTTTACTAGTGCCATTTGTTCAGTGGTT CGTAGGGCTTTCCCCCACTGTCTGGCTTTCAGTTATATGGATGATGTGGTtTTGGGGGCC AAGTCTGTACAACATCTTGAGTCCCTTTATGCCGCTGTTACCAATTTTCTTTTGTCTTTG GGTATACATTTAAACCCTCAcAAAACAAAAAGATGGGGATATTCCCTTAACTTCATGG GATATGTAATTGGGAGTTGGGGCACaTTGCCaCAGGAACATATTGTACAAAAAATCAA AaTgTGTTTTAGGAAACTTCCTGTAAACAGGCCTATTGATTGGAAAGTATGTCAACGAA TTGTGGGTCTTTTGGGGTTTGCcGCCCCTTTcACgCAATGTGGATATCCTGCTTTAATGC CTTTATATGCATGTATACAAGCaAAACAGGCTTTTACTTTCTCGCCAACTTACAAGGCC TTTCTAAGTAAACAGTATCTGAACCTTTACCCCGTTGCTCGGCAACGGCCTGGTCTGTG CCAAGTGTTTGCTGACGCAACCCCCACTGGTTGGGGCTTGGCCATAGGCCATCAGCGC ATGCGTGGAACCTTTGTGTCTCCTCTGCCGATCCATACTGCGGAACTCCTAGCCGCTTG TTTTGCTCGCAGCaGGTCTGGgGCaAAACTcATCGGGACTGACAATTCTGTCGTgCTcTCC CGCAAGTATACATCaTTTCCATGGCTGCTAGGCTGTGCTGCCAACTGGATCCTGCGCGG GACGTCCTTTGTTTACGTCCCGTCGGCGCTGAATCCCGCGGACGACCCCTCCCGGGGCC GCTTGGGGCTCTACCGCCCGCTTCTCCGcCTGttGTACCGACCGACCACGGGGCGCACCT CTCTTTACGCGGACTCCCCGTCTGTGCCTTCTCATCTGCCGGACCGTGTGCACTTCGCTT CACCTCTGCACGTCGCATGGAGACCACCGTGAACGCCCACcGGAACCTGCCCAAGGTC TTGCATAAGAGGACTCTTGGACTTTCAGCAATGTCAACGACCGACCTTGAGGCATACT TCAAAGACTGTGTGTTTAcTGAGTGGAGGAGTTGGGGGAGGAGaTTAGGTTAAAGGT CTTTGTACTAGGAGGCTGTAGGCATAAATTGGTgTGTTCACCAGCACCATGCAACTTTT TCACCTCTGCCTAATCATCTCaTGTTCATGTCCTACTGTTCAAGCCTCCAAGCTGTGCCT TGGGTGGCTTTgGGGCATGGACATTGACCCGTATAAAGAATTTGGAGCTTCTGTGGAGT TACTCTCTTTTTTGCCTTCTGACTTCTTTCCTTCTATTCGAGATCTcCTCGACACCGCCTC TGCTCTGTATCGGGAGGCCTTAGAGTCTCCGGAACATTGTTCACCTCACCATACGGCAC TCAGGCAAGCTATTcTGTGTTGGGGTGAGTTgATGAATCTAGCCACCTGGGTGGGAAGT AATTTGGAAGATCCAGCATCCAGGGAATTAGTAGTCAGCTATGTCAAcGTTAATATGG GCCTAAAAATCAGACAACTATTGTGGTTTCACATTTCCTGTCTTACTTTTGGgAGAGAA |
| ACTGTTCTTGAATATTTGGTGTCTTTTGGAGTGTGGATTCGCACTCCTCCTGCATATAG ACCACCAAATGCCCCTATCTTATCAACACTTCCGGAAACTACTGTTGTTAGACGAaGAG GCAGGTCCCCTAGAAGAAGAACTCCCTCGCCTCGCAGACGAAGGTCTCAATCGCCGCG TCGCAGAAGATCTCAATCTCGGGAATCTCAATGTTAGTATTCCTTGGACaCATAAGGTG GGAAACTTTACGGGGCTTTATTCTTCTACGGTACCTtGCTTTAATCCTaAaTGGCAAACT CCTTCTTTTCCTGACATTCATTTGCAGGAGGACATTGTTGATAGaTGTAAGCAATTTGTG GGgCCCCTtACAGTAAATGAAAACAGGAGACTAAAATTAATTATGCCTGCTAGGTTTTA TCCCAATGTTACTAAATATTTGCCCTTAGATAAAGGgATCAAACCgTATTATCCAGAGc ATGTAGTTAATCATTACTTCCAGACGaGACATTATTTACAcACTCTTTGGAAGGCGGGtA TCTTATATAAAAGAGAGtCcACACGTAGCGCCTCATTTTGCGGGTCACCATATTCTTGG GAACAAGAtCTACAGCATGGGAGGTTGGTCTTCCAAACCTCGAAAAGGCATGGGGACA AATCTTTCTGTCCCCAATCCcCTGGGATTCTTCCCCGATCATCAGTTGGACCCTGCATTC AAAGCCAACTCAGAAAATCCAGATTGGGACCTCAACCCgCACAAGGACAACTGGCCG GACGCCAACAAGGTGGGAGTGGGAGCATTCGGGCCAGGGTTCACcCCTCCCCATGGGG GACTGTTGGGGTGGAGCCCTCAGGCTCAGGGCaTACTCACAACTGTGCCAGCAGCTCC TCCTCCTGCCTCCACCAATCGGCAGTCAGGAAGGCAGCCTACTCCCtTATCTCCACCTC TAAGGGACACTCATCCTCAGGCCATGCAGTGGAA |
| **virtual HBV C genotype sequence (****FIG. 3****) SEQ ID NO. 92** |
| CTCCAcaACATTCCACCAAGCTCTGCTAGAtCCCAGAGTGAGGGGCCTATAcTTTCCTGC TGGTGGCTCCAGTTCCGGAACAGTAAACCCTGTTCCGACTACTGCCTCaCCCATATCGT CAATCTTCTCGAGGACTGGGGACCCTGCACCGAACATGGAGAaCACaACATCAGGATT CCTAGGACCCCTGCTCGTGTTACAGGCGGGGTTTTTCTTGTTGACAAGAATCCTCACAA TACCACAGAGTCTAGACTCGTGGTGGACTTCTCTCAATTTTCTAGGGGGAGCACCCAC GTGTCCTGGCCAAAATTcGCAGTCCCCAACCTCCAATCACTCACCAACCTCTTGTCCTC CAATTTGTCCTGGcTATCGCTGGATGTGTCTGCGGCGTTTTATCATaTTCCTCTTCATCCT GCTGCTATGCCTCATCTTCTTGTTGGTTCTTCTGGACTACCAAGGTATGTTGCCCGTTTG TCCTCTACTTCCAGGAACATCAACTACCAGCACGGGACCATGCAAGACCTGCACGAtTC CTGCTCAAGGAACCTCTATGTTTCCCTCTTGTTGCTGTACAAAACCTTCGGACGGAAAc |
| TGCACTTGTATTCCCATCCCATCATCcTGGGCTTTCGCAAGATTCCTATGGAGTGGGC CTCAGTCCGTTTCTCCTGGCTCAGTTTACTAGTGCCATTTGTTCAGTGGTTCGTAGGGCT TTCCCCCACTGTTTGGCTTTCAGTTATATGGATGATGTGGTATTGGGGGCCAAGTCTGT ACAACATCTTGAgTCCCTTTTTACCTCTATTACCAATTTTCTTTTGTCTTTGGGTATACAT TTgAACCCTAATAAAACCAAACGTTGGGGCTACTCCCTTAACTTCATGGGATATGTAAT TGGAAGTTGGGGTACtTTaCCaCAgGAACATATTGTACtAAAAaTCAAgCAATGTTTTCGa AAACTgCCTgTAAATAGACCTATTGATTGGAAAGTATGTCAaAGAATTGTGGGTCTTtTG GGCTTTGCTGCCCCTTTTACACAATGTGGcTAtCCtGCcTTaATGCCTTTaTATGCATGTAT ACAAtCTAAGCAGGCTTTCACTTTCTCGCCAACTTACAAGGCCTTTCTGTGTAAACAAT ATCTGAACCTTTACCCCGTTGCcCGGCAACGGTCAGGTCTcTGCCAAGTGTTTGCTGAC GCAACCCCCACTGGaTGGGGCTTGGCcATaGGCCATCggCGCATGCGTGGAACCTTTGTG GCTCCTCTGCCGATCCATACTGCGGAACTCCTAGCAGCTTGTTTTGCTCGCAGCCGGTC TGGAGCgAAACTTATCGGaACcGACAACTCTGTTGTCCTCTCTCGGAAATACACCTCCTT TCCATGGCTGCTaGGgTGTGCTGCCAACTGGATCCTGCGCGGGACGTCCTTTGTCTACG TCCCGTCGGCGCTGAATCCCGCGGACGACCCGTCTCGGGGCCGTTTGGGaCTCTACCGT CCCCTTCTTCaTCTGCCGTTCCGGCCGACCACGGGGCGCACCTCTCTTTACGCGGTCTCC CCGTCTGTGCCTTCTCATCTGCCGGACCGTGTGCACTTCGCTTCACCTCTGCACGTCGC ATGGAgACCACCGTGAACGCCCaCCAGGTCTTGCCCAAGGTCTTACATAAGAGGACTCT TGGACTCTCAGCAATGTCAACGACCGACCTTGAGGCATACTTCAAAGACTGTtTgTTTA AaGACTGGGAGGAGTTGGGGGAGGAGATTAGGTTAAaGaTCTTTGTACTaGGAGGCTGT AGGCATAAATTGGTCTGTTCACCAGCACCATGCAACTTTTTCACCTCTGCCTAATCATC TCATGTTCATGTCCTACTGTTCAAGCCTCCAAGCTGTGCCTTGGGTGGCTTTgGGGCAT GGACATTGACCCGTATAAAGAATTTGGAGCTTCTGTGGAGTTACTCTCTTTTTTGCCTT CTGACTTCTTTCCTTCTATTCGAGATCTCCTCGACACCGCCTCtGCTCTGTATCGGGAGG CCTTAGAGTCTCCGGAACATTGTTCACCTCACCATACAGCACTcAGGCAAGCTATTCTG TGTTGGGGTGAGTTGATGAATCTGGCCACCTGGGTGGGAAGTAATTTGGAAGACCCAG CATCCAGGGAATTAGTAGTcAGCTATGTCAATGTTAATATGGGCCTAAAAATCAGACA ACTAtTGTGGTTTCACATTTCCTGTCTTACTTTTGGAAGAGAAACTGTTCTTGAGTATTT |
| GGTGTCTTTTGGAGTGTGGATTCGCACTCCTcCcGCTTACAGACCACCAAATGCCCCTAT CTTATCAACACTTCCGGAAACTACTGTTGTTAGACGACGAGGCAGGTCCCCTAGAAGA AGAACTCCCTCGCCTCGCAGACGAAGGTCTCAATCGCCGCGTCGCAGAAGATCTCAAT CTCGGGAATCTCAATGTTAGTATCCCTTGGACTCATAAGGTGGGAAACTTTACTGGGCT TTATTCTTCTACTGTACCTGTCTTTAATCCTGAgTGGCAAACTCCCTCcTTTCCTcACATT CATTTacAgGAGGAcATTATtAATAGATGTCAACAATATGTGGGCCCTCTTACAGTTAAT GAAAAAAGGAGATTAAAATTAATTATGCCTGCTAGGTTCTATCCTAACCTTACCAAAT ATTTGCCCTTgGAcAAAGGCATTAAACCtTATTATCCTGAACATgCAGTTAATCATTACT TCaAAACTAGGCATTATTTACATACTCTGTGGAAGGCtGGcATTcTATATAAGAGAGAAA CTACACGCAGCGCCTCATTTTGTGGGTCACCATATTCTTGGGAACAAGAGCTACAGCA TGGGAGGTTGGTCTTCCAAACCTCGACAAGGCATGGGGACGAATCTTTCTGTTCCCAA TCCTCTGGGATTCTTTCCCGATCACCAGTTGGACCCtGCGTTCGGAGCCAAcTCAAACA ATCCAGATTGGGACTTCAACCCCAACAAGGATCAcTGGCCAGaGGCAAAtCAGGTAGGA GcGGGAGCATTCGGGCCAGGGTTCACcCCACCACACGGCgGTCTTTTGGGGTGGAGCCC TCAGGCTCAGGGCATATTGACAACAGTGCCAGcAGCaCCTCCTCCTGCCTCCACCAATC GGCAGTCAGGAAGACAGCCTACTCCCATCTCTCCACCTCTAAGAGACAGTCATCCTCA GGCCATGCAGTGGAA |
| **virtual HBV D genotype sequence (****FIG. 4****) SEQ ID NO. 93** |
| CTCCACAACCTTCCACCAAACTCTGCAAGATCCCAGaGTGAGAGGCCTGTATTTCCCTG CTGGTGGCTCCAGTTCAGGAACAGTAAACCCTGTTCCGACTACTGtCTCTCcCATATCGT CAATCTTCTCGAGGATTGGGGACCCTGCGCTGAACATGGAGAACATCACATCAGGATT CCTAGGACCCCTGCTCGTGTTACAGGCGGGGTTTTTCTTGTTGACAAGAATCCTCACAA TACCGCAGAGTCTAGACTCGTGGTGGACTTCTCTCAATTTTCTAGGGGGAACtACCGTG TGTCTTGGCCAAAATTCGCAGTCCCCAACCTCCAATCACTCACCAACCTCCTGTCCTCC AACTTGTCCTGGTTATCGCTGGATGTGTCTGCGGCGTTTTATCATCTTCCTCTTCATCCT GCTGCTATGCCTCATCTTCTTGTTGGTTCTTCTGGACTATCAAGGTATGTTGCCCGTTTG TCCTCTAATTCCAGGATCtTCAACCACCAGCaCGGGACCATGCAGAACCTGCACGACTc |
| CTGCTCAAGGAACCTCTATGTATCCCTCCTGTTGCTGTACCAAACCTTCGGACGGAAAT TGCACCTGTATTCCCATCCCATCATCCTGGGCTTTCGGAAAATTCCTATGGGAGTGGGC CTCAGCCCGTTTCTCCTGGCTCAGTTTACTAGTGCCATTTGTTCAGTGGTTCGTAGGGC TTTCCCCCACTGTTTGGCTTTCAGTTATATGGATGATGTGGTATTGGGGGCCAAGTCTG TACAGCATCTTGAGTCCCTTTTTACCGCTGTTACCAATTTTCTTTTGTCTTTGGGTATAC ATTTAAACCCTAACAAAACaAAAGATGGGGTTACTCTtTAcATTTcATGGGcTATGTCA TTGGATGTTATGGGTCaTTGCCACAAGATCACATCATACAgAAAATCAAAGAATGTTTT AGAAAACTTCCTGTTAACAGGCCTATTGATTGGAAAGTcTGTCAACGtATTGTGGGTCT TTTGGGTTTTGCTGCCCCTTTTACACAATGTGGTTATCCTGCTTTAATGCCcTTGTATGCa TGTATTCAATCTAAGCAGGCTTTCACTTTCTCGCCAACTTACAAGGCCTTTCTGTGTAA ACAATACCTGAACCTTTACCCCGTTGCCCGGCAACGGCCAGGTCTGTGCCAAGTGTTT GCTGACGCAACCCCCACTGGCTGGGGCTTGGTCATGGGCCATCAGCGCATGCGTGGAA CCTTTCtGGCTCCTCTGCCGATCCATACTGCGGAACTCCTAGCCGCTTGTTTTGCTCGCA GCAGGTCTGGAGCAAACATTcTCGGGACgGATAACTCTGTTGTTCTCTCCCGCAAATAT ACATCGTTTCCATGGCTGCTAGGCTGTGCTGCCAACTGGATCCTGCGCGGGACGTCCTT TGTTTACGTCCCGTCGGCGCTGAATCCCGCGGACGACCCTTCTCGGGGcCGCTTGGGAC TCTCTCGTCCCCTTCTCCGTCTGCCGTTtCGaCCGACCACGGGGCGCACCTCTCTTTACG CGGACTCCCCGTCTGTGCCTTCTCATCTGCCGGACCGTGTGCACTTCGCTTCACCTCTG CACGTCGCATGGAGACCACCGTGAACGCCCACCAatTCTTGCCCAAGGTCTTACATAAG AGGACTCTTGGACTCTCTGtAATGTCAACGACCGACCTTGAGGCATACTTCAAAGACTG TTTGTTTAAaGACTGGGAGGAGTTGGGGGAGGAGATTAGaTTAAaGgTCTTTGTAcTAGG AGGCTGTAGGCATAAATTGGTCTGCGCACCAGCACCATGCAACTTTTTCACCTCTGCCT AATCATCTCTTGTTCATGTCCTACTGTTCAAGCCTCCAAGCTGTGCCTTGGGTGGCTTTg GGgCATGGACATTGAcCCTTATAAAGAATTTGGAGCTaCTGTGGAGTTACTCTCGTTTTT GCCTTCTGACTTCTTTCCTTCAGTACGAGATCTTCTAGATACCGCCTCAGCTCTgTATCG GGAAGCCTTAGAGTCTCCTGAGCATTGTTCACCTCACCATACTGCACTCAGGCAAGCA ATTCTtTGCTGGGGGGAaCTAATGACTCTAGCtACCTGGGTGGGTGgTAATTTGGAAGAT CCAgcATCcAGGGACCTAGTAGTCAGTTATGTcAACACTAATATGGGCCTAAAGTTCAG |
| GCAACTaTTGTGGTTTCACATTTCTTGTCTCACTTTTGGAAGAGAAACgGTcaTAGAGTA TTTGGTGTCTTTCGGAGTGTGGATTCGCACTCCTCCAGCTTATAGACCACCaAATGCCC CTATCTTATCAACACTTCCGGAGACTACTGTTGTTAGACGACGAGGCAGGTCCCCTAG AAGAAGAACTCCCTCGCCTCGCAGACGAAGGTCTCAATCGCCGCGTCGCAGAAGATCT CAATCTCGGGAATCTCAATGTTAGTATTCCTTGGACTCATAAGGTGGGaAACTTTACGG GGCTTTATTCTTCTACTGTaCCTGTCTTTAACCCTCATTGGAAAACACCcTCTTTTCCTAA TATACATTTACACCAAGACATTATCAAAAAATGTGAACAaTTTGTAGGCCCACTCACAG TcAATGAGAAAaGAAGAcTGCAATTgATTATGCCTGCTAGGTTTTATCCAAAtGTTACCA AATATTTGCCATTgGATAAGGGTATTAAACCTTATTATCCAGAACATCTAGTTAATCAT TACTTCCAAACcAGACAtTATTTACACACTCTATGGAAGGCGGGTATATTATATAAGAG AGAAACAACACaTAGCGCCTCATTTTGTGGGTCACCATATTCTTGGGAACAAGAGCTA CAGCATGGGGCAGAATCTTTCCACCAGCAATCCTCTGGGATTCTTTCCCGACCACCAGT TGGATCCAGCCTTCAGAGCAAACACCGCAAATCCAGATTGGGACTTCAATCCCAACAA GGACACCTGGCCAGACGCCAACAAGGTAGGAGCTGGAGCATTCGGGCTGGGaTTCACC CCACCGCACGGAGGCCTTTTGGGGTGGAGCCCTCAGGCTCAGGGCATAcTACAAACcTT GCCAGCAAATCCGCCTCCTGCcTCtACCAATCGCCAGTCAGGAAGGCAGCCTACCCCgC TGTCTCCACCTTTGAGAAACACTCATCCTCAGGCCATGCAGTGGAA |

### Example 2. Guide RNA (gRNA) design

CRISPR target gRNAs were designed based on virtual universal HBV genotypes B, C and D obtained in Example 1, respectively.

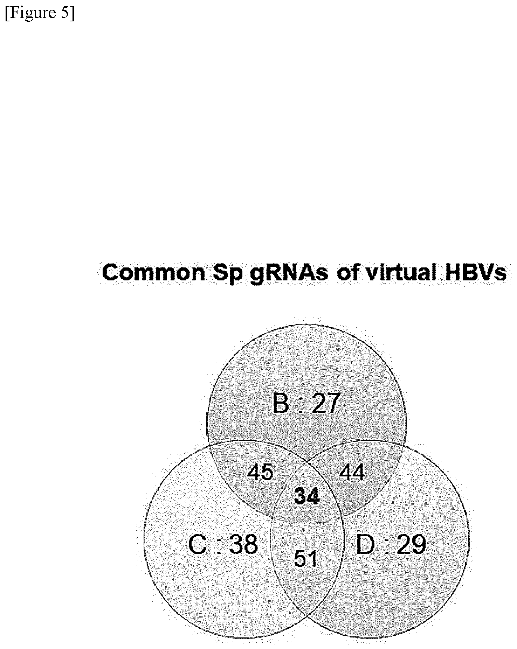

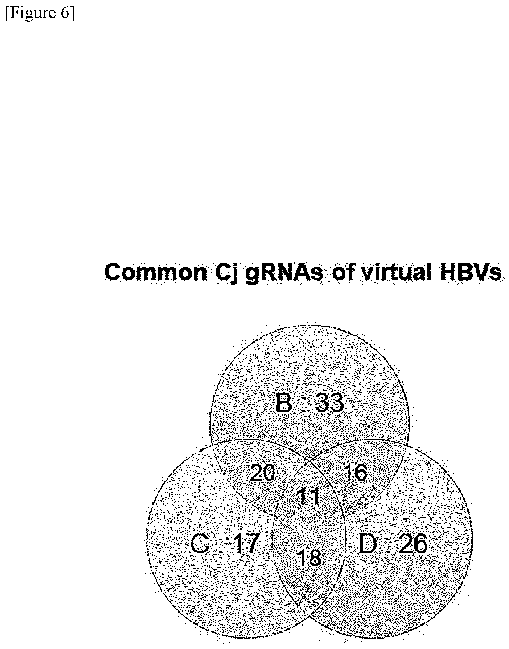

gRNAs for SpCas9 (from *Streptococcus pyogenes* (5'-NGG-3' for target, 5'-NRG-3' for off-target)) and CjCas9 (from *Campylobacter jejuni:* 5'-NNNNRYAC-3') were designed with Cas-Designer(http://www.rgenome.net/cas-designer/), and gRNAs having off-target sequences with 1 and 2 mismatches were excluded. The gRNAs conserved in vHBV genotypes B, C and D are obtained as shown in FIGS. 5 and 6.

As a result of designing, targets (150 gRNAs for SpCas9 and 80 gRNAs for CjCas9) for the virtual universal HBV genotype B, targets (168 gRNAs for SpCas9 and 66 gRNAs for CjCas9) for HBV genotype C and targets (158 gRNAs for SpCas9, 71 gRNAs for CjCas9) for HBV genotype D were obtained. In addition, 34 gRNAs for SpCas9, which were able to target all of the virtual universal HBV genotypes B, C and D, were obtained, and 11 gRNAs for CjCas9, which were able to target all of the virtual universal HBV genotypes B, C and D, were obtained (FIGS. 5 and 6).

The gRNAs of SEQ ID NOs. 46 to 90 in Table 2 are gRNAs capable of targeting at least one of the HBV genotypes B, C and D, and among these, SEQ ID NOs. 46 to 79 are gRNAs for SpCas9, and SEQ ID NOs. 80 to 90 are gRNAs for Cj Cas9.

### Example 3. Selection of gRNAs targeting all of HBV genotypes B, C and D

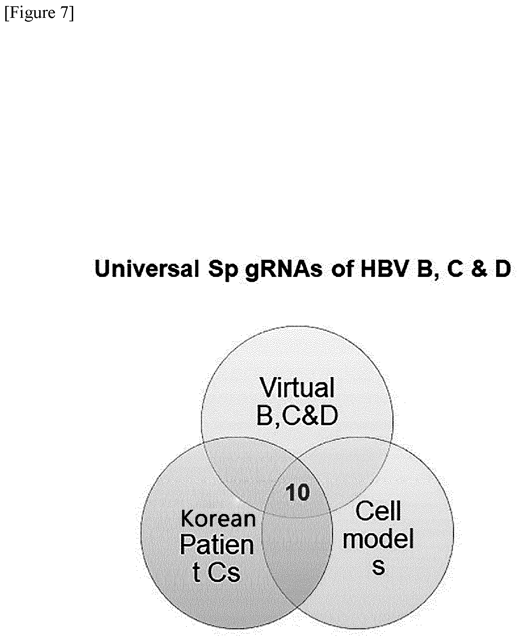

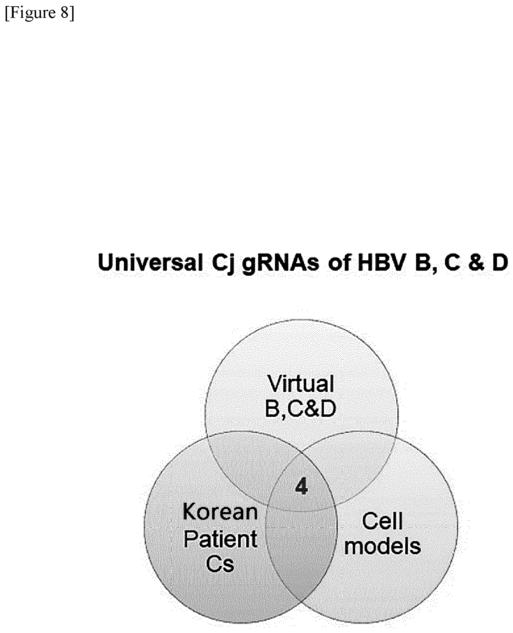

As gRNAs targeting a common conserved region in a sequence targeting open database-based conserved, Korean patient-derived HBV genotype C and a sequence of an available HBV cell model, 10 gRNAs for SpCas9 and 4 gRNAs for CjCas9 were designed (FIGS. 7 and 8).

gRNAs of SpCas9 capable of targeting all of four viruses, which are patient-derived HBV type C, and two cell lines (type D) generally used in an HBV experiment are SEQ ID NOs. 51, 52, 56, 57, 69, 70, 73, 74, 75 and 76 in Table 2, and gRNAs of CjCas9 capable of targeting all of four viruses, which are patient-derived HBV type C, and two cell lines (type D) generally used in an HBV experiment are SEQ ID NOs. 80, 85, 87 and 89 in Table 2.

### Example 4. Screening of HBV target region

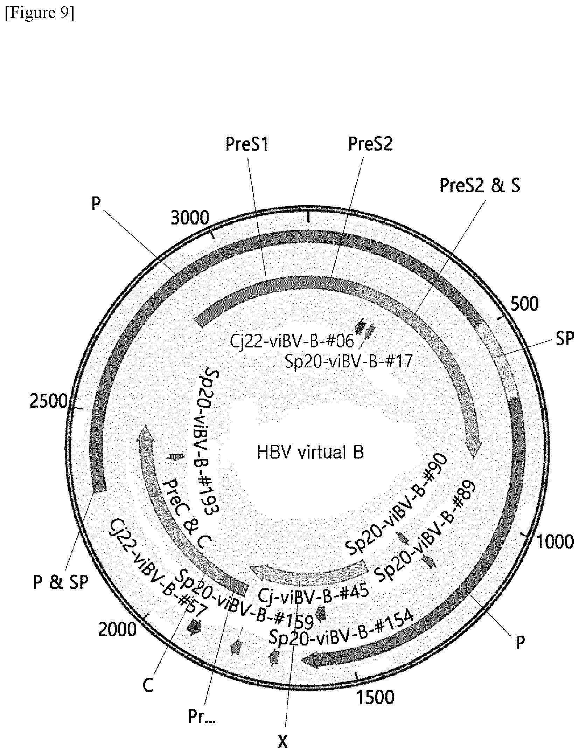

As a result of screening an HBV target region targeting gRNAs targeting the common conserved region in the open database-based conserved sequence, Korean patient-derived HBV genotype C and an HBV cell model, which were obtained in Example 3, it was confirmed that one or more of HBV P, X, PreS1, PreS2, C, S and PreC of FIG. 9 are targeted.

The HBV target region targeted by each of the above gRNAs is shown in Table 4.

**[Table 4]**

| **NO.** | **Name** | **Note.** | **cccDNA target region** |
|---|---|---|---|
| **SEQ ID NO. 51** | Sp20-viHBV-B-#17G | SpCas9 target | P, PreS1, PreS2, S |
| **SEQ ID NO. 52** | Sp20-viHBV-B-#20G | | P, PreS1, PreS2, S |
| **SEQ ID NO. 56** | Sp20-viHBV-B-#89G | | P |
| **SEQ ID NO. 57** | Sp20-viHBV-B-#90G | | P |
| **SEQ ID NO. 69** | Sp20-viHBV-B-#154G | | X |
| **SEQ ID NO. 70** | Sp20-viHBV-B-#159G | | X |
| **SEQ ID NO. 73** | Sp20-viHBV-B-#193G | | P, SP, PreC, C |
| **SEQ ID NO. 74** | Sp20-viHBV-B-# 194G | | P, SP, PreC, C |
| **SEQ ID NO. 75** | Sp20-viHBV-B-# 196G | | P, SP, PreC, C |
| **SEQ ID NO. 76** | Sp20-viHBV-B-#197G | | P, SP, PreC, C |
| **SEQ ID NO. 80** | Cj22-viHBV-B-#06G | CjCas9 target | P, PreS1, PreS2, S |
| **SEQ ID NO. 85** | Cj22-viHBV-B-#45G | | P, X |
| **SEQ ID NO. 87** | Cj22-viHBV-B-#47G | | P, X |
| **SEQ ID NO. 89** | Cj22-viHBV-B-#57G | | PreC |

### Example 5. Cell culture and transfection

### i) Vectors

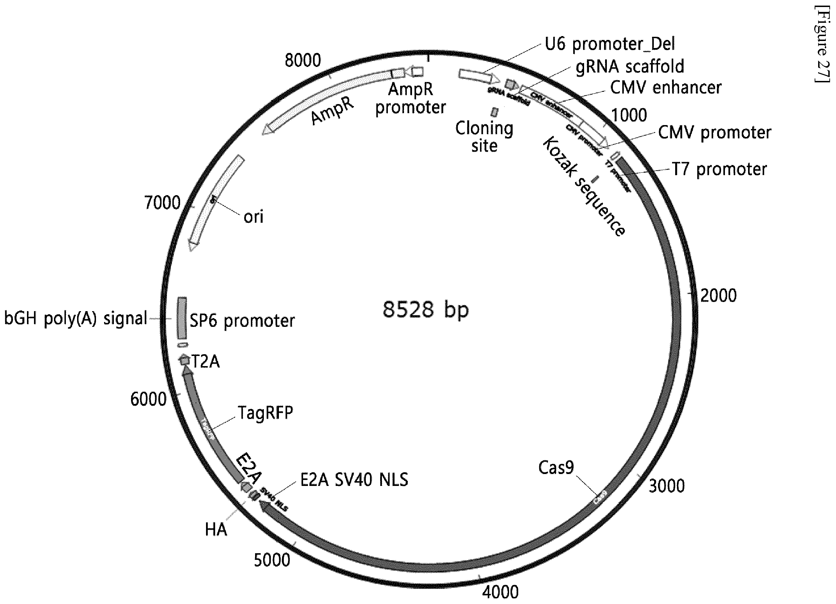

Vector construction for introducing the gRNAs obtained in Example 3 into cells was prepared (FIGS. 27 to 29).

200 pmol each of sense and antisense oligonucleotides of gRNA was mixed with a T4 ligase buffer, and subjected to oligo annealing under conditions including heating at 95 °C for 2 minutes, 5 cycles of cooling from 95 to 85 °C at -2 °C/s, and 60 cycles of cooling from 85 to 25 °C at -1 °C/s, followed by insertion behind the U6 promoter of a plasmid using a restriction enzyme.

In FIG. 27, one SpgRNA, Cas9 and a fluorescent protein were designed to be expressed in one plasmid.

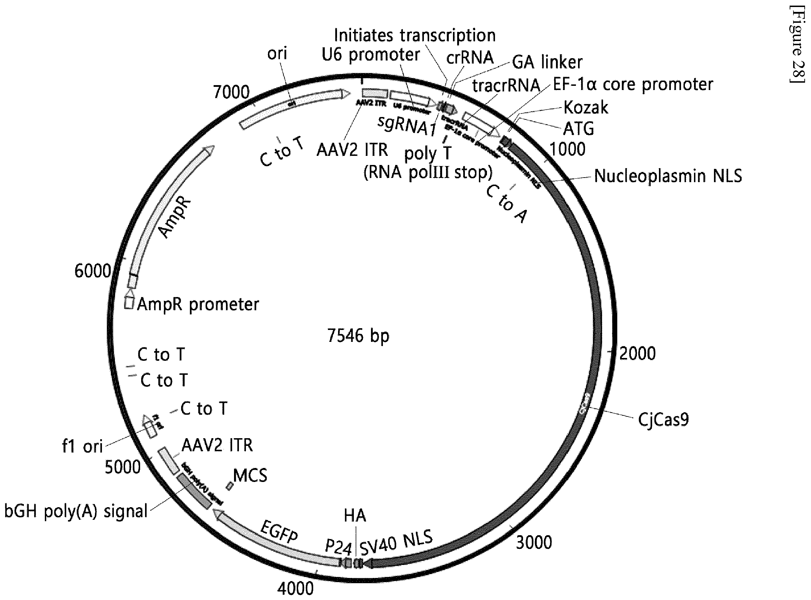

In FIG. 28, one CjgRNA, Cas9 and a fluorescent protein were designed to be expressed in one plasmid.

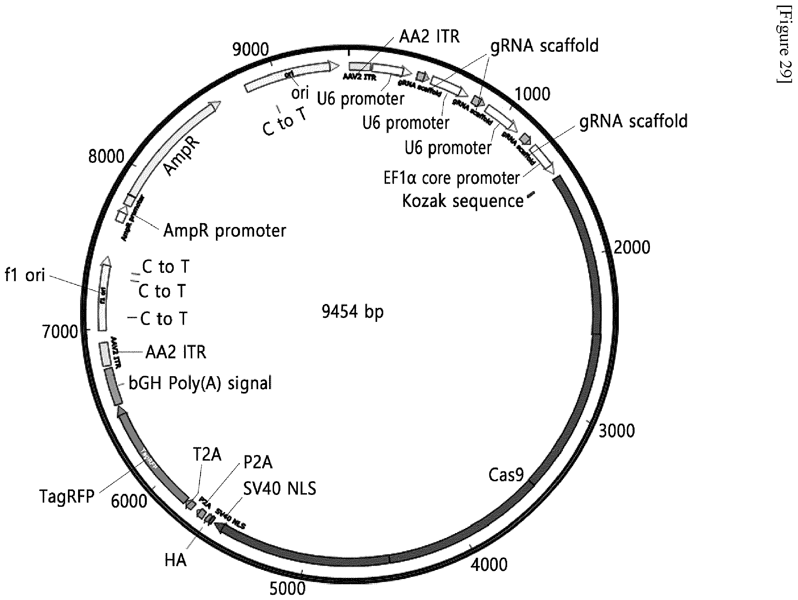

In FIG. 29, three SpgRNAs, Cas9 and a fluorescent protein were designed to be expressed in one plasmid.

### ii) Materials

The HBV 1.2 plasmid carrying 1.2-copies of the HBV genome (genotype D, Addgene #51294); pAAV HBV genotypes A, B and C (kindly provided by Pei-Jer Chen, Taipei City, Taiwan); Cj CRISPR Cas9 gRNA plasmids (Cj#06, Cj#45, Cj#47, Cj#57) (Toolgen); and Sp CRISPR Cas9 gRNA plasmids (Sp#17, Sp#20, Sp#89, Sp#90, Sp#154, Sp#159, Sp#193, Sp#194, Sp#196, Sp#197, N#17, N#90, N#193, N#197, N#17-90-193, N#17-90-197, N#17-193-197, N#90-193-197) (Toolgen).

Human liver cancer cell lines Huh7 and HepG2 were obtained from the Korean Cell Line Bank (Seoul, Korea). For cell culture and maintenance, 10% fetal bovine serum (Capricorn, Ebsdorfergrund, Germany) and 1% penicillin/streptomycin (Gibco, Carlsbad, CA, USA) were added to Dulbecco's modified Eagle's medium (Welgene, Gyeongsan-si, Korea). The cells were maintained in an incubator at 37 °C with 5% CO₂.

4 x 10⁵ of the Huh7 or HepG2 cells were seeded in each well of a 6-well plate, and 1 µg of a replicon and 1 µg of a plasmid expressing CRISPR Cas9 (SpCas9 or CjCas9) and gRNA were transfected using Lipofectamine 2000. After 24 hours, the cell medium was replaced with a fresh medium. After three days, a cell supernatant was obtained for ELISA.

### Example 6. Measurement of HBsAg and HBeAg

To measure HBsAg and HBeAg levels in the cell supernatant obtained in Example 5, as commercial ELISA kits, HBeAg ELISA Kit (WB-2496, Wantai Pharm Inc., Beijing, China) and HBsAg ELISA Kit (WB-2296, Wantai Pharm Inc., Beijing, China) were used, and the measurement was performed according to the protocol of the kit. The cell supernatant was used by diluting HBeAg to 1/10 and HBsAg to 1/25 with PBS. The absorbance of HBeAg and HBsAg was measured in a wavelength range of 450 nm using the SPECTRA max PLUS 384 Microplate Reader and the SoftMax Pro 5.2 program.

### Result 1) Screening of SpCas9 gRNA candidate and CjCas9 target gRNA candidate

Each of the SpCas9 gRNA candidates and CjCas9 gRNA candidates obtained in Example 3 was used to measure HBeAg and HBsAg levels using HBV D genotype HBV 1.2.

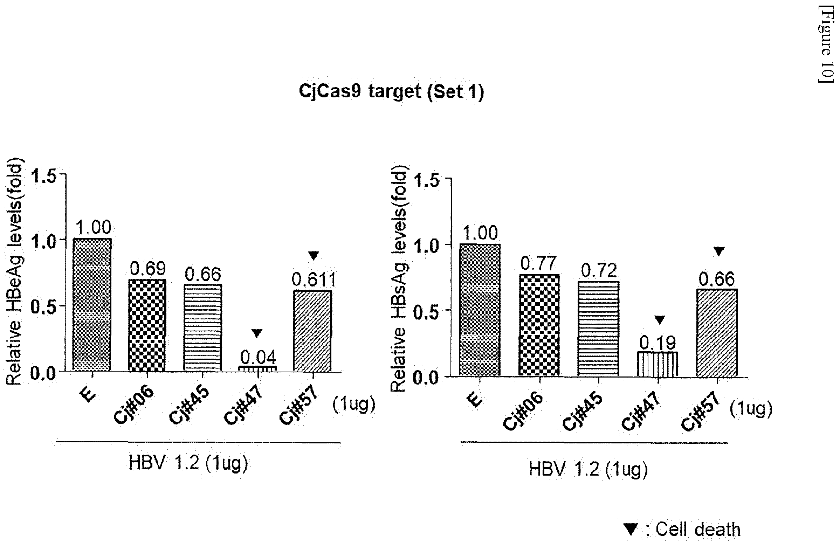

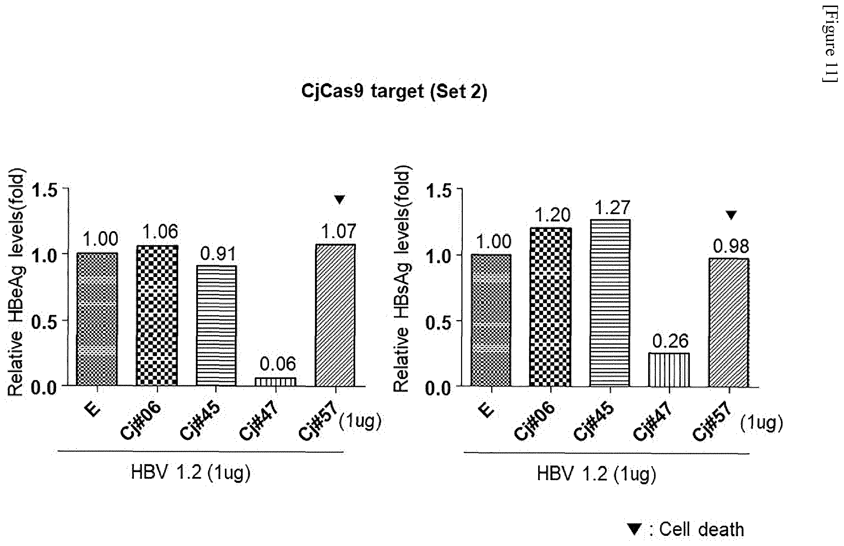

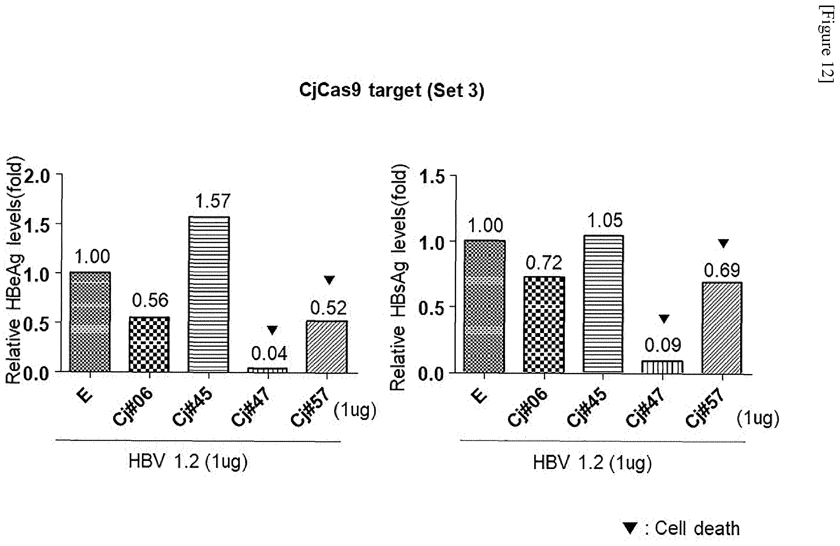

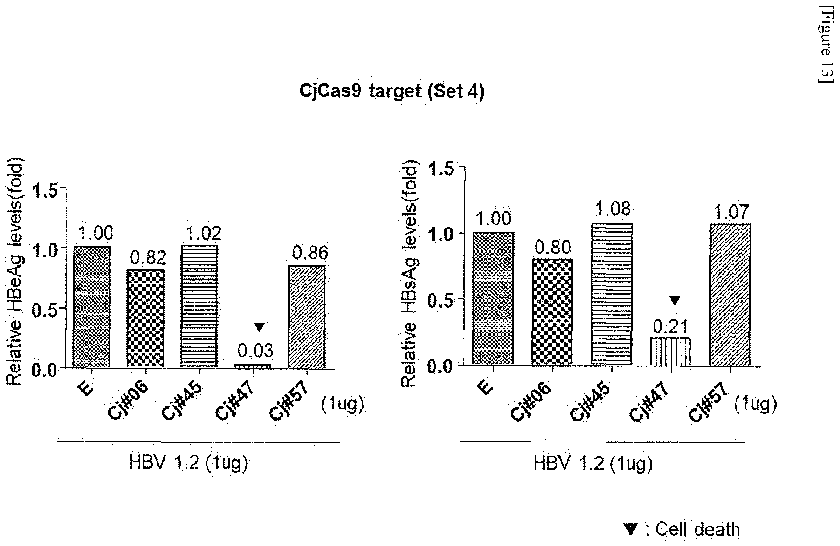

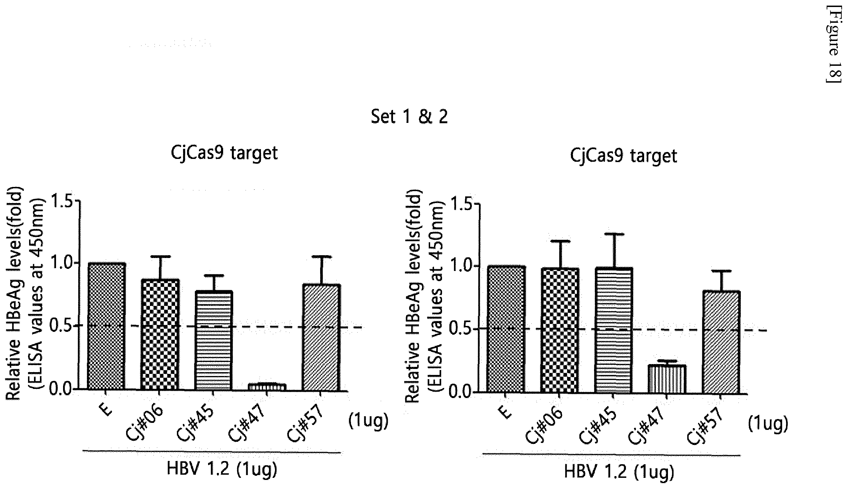

As a result of measuring HBeAg and HBsAg levels of each of the CjCas9 gRNA candidates such as Cj#06, Cj#45, Cj#47 and Cj#57, it can be seen that Cj#47 had the lowest levels of HBeAg and HBsAg (FIGS. 10 to 13, and FIG. 18). That is, it was expected that Cj#47 is CjCas9 target gRNA having the greatest effect.

As a result of measuring HBeAg and HBsAg levels of each of the SpCas9 gRNA candidates such as Sp#17, Sp#20, Sp#89, Sp#90, Sp#154, Sp#159, Sp#193, Sp#194, Sp#196 and Sp#197, most cases had low HBeAg levels, but relatively high HBsAg levels.

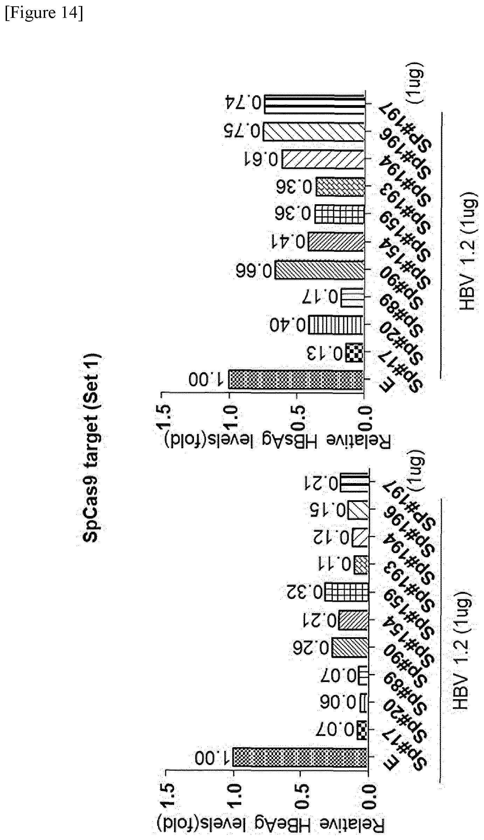

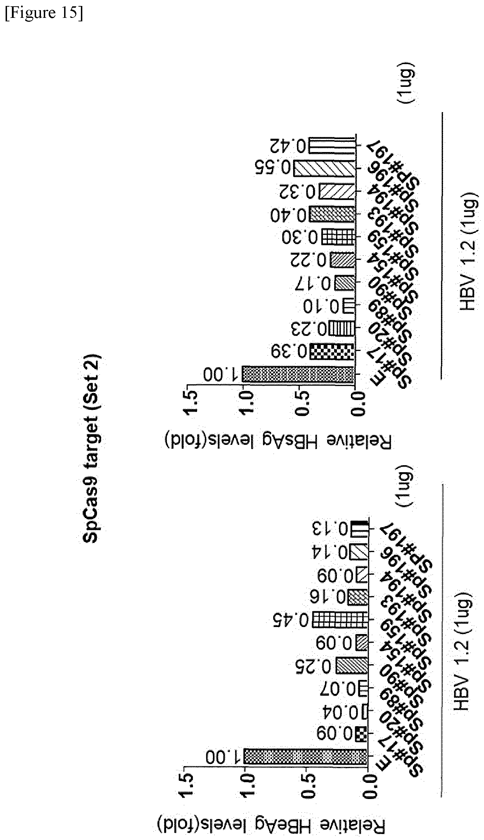

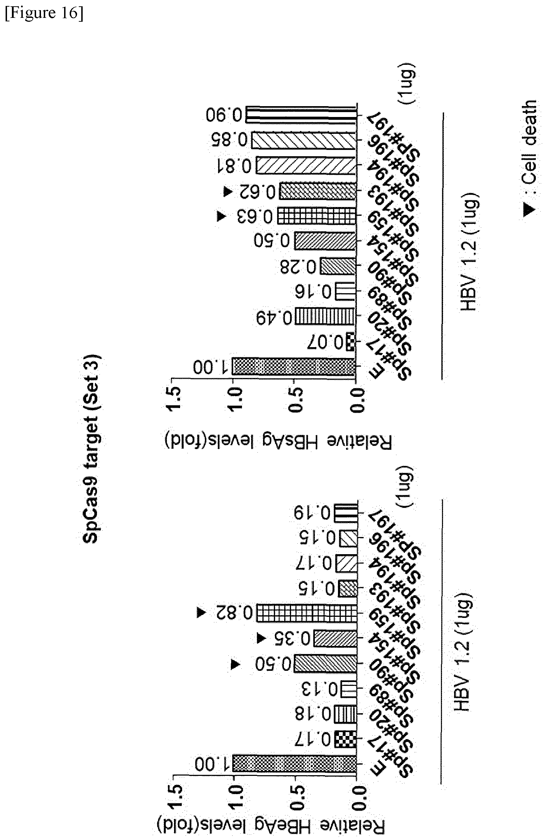

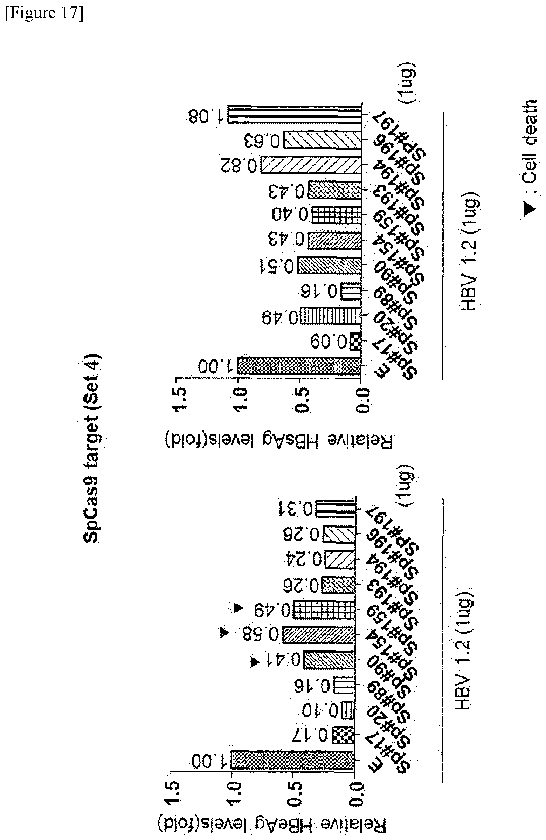

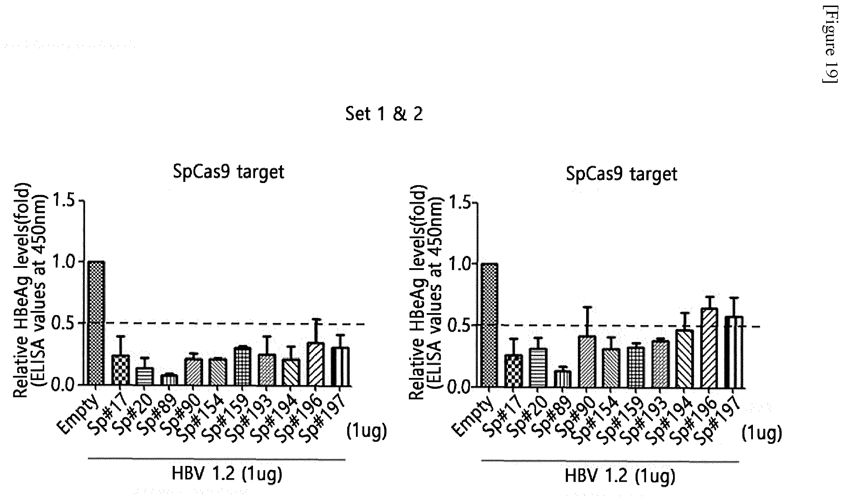

When the HBeAg and HBsAg levels of the various gRNA candidates were measured, it was found that the degrees of HBeAg and HBsAg inhibition for each candidate were different (FIGS. 14 to 17, and FIG. 19).

That is, it can be confirmed that even gRNAs targeting the conserved sequence of hepatitis B virus had different HBeAg and HBsAg inhibitory capacities.

### Result 2) Confirmation of HBeAg and HBsAg inhibitory capacity in HBV cell model (HBV type D) of SpCas9 gRNA candidates

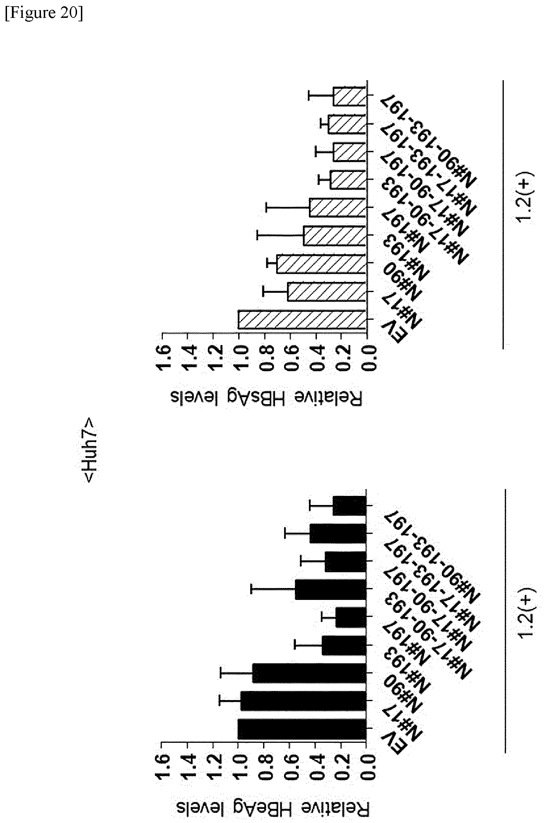

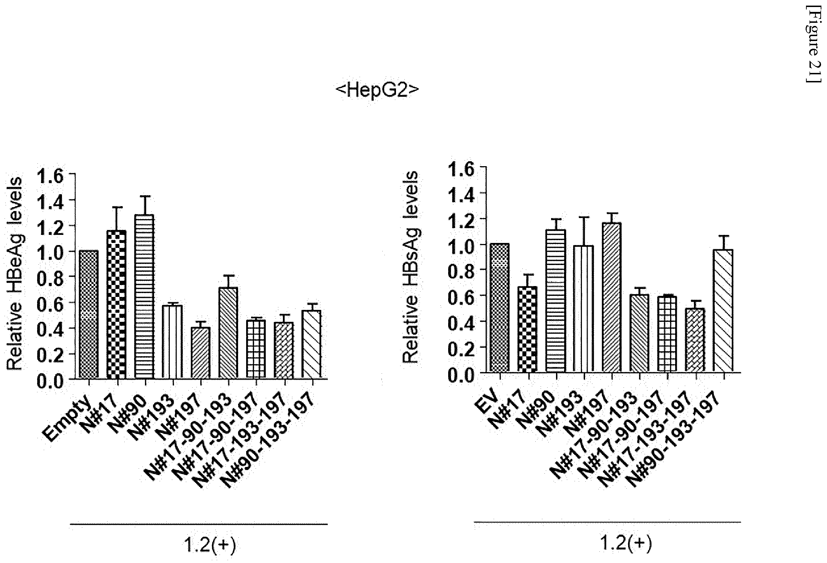

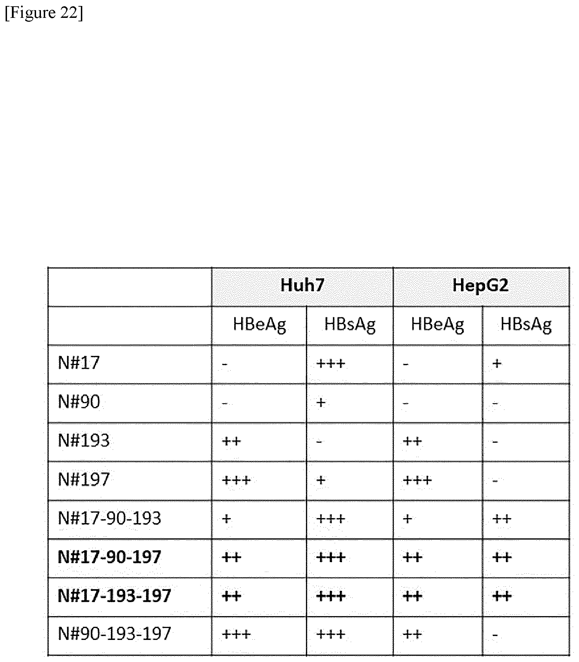

In each of human liver cancer cell lines Huh7 (pAAV HBV D genotype-introduced cells) and HepG2 (pAAV HBV D genotype-introduced cells), HBeAg and HBsAg levels for Sp#17 (named N#17), Sp#90 (named N#90), Sp#193 (named N#193), Sp#197 (named N#197), Sp#17+Sp#90+Sp#193 combination (named N#17-90-193), Sp#17+Sp#90+Sp#197 combination (named N#17-90-197), Sp#17+Sp#193+Sp#197 combination (named N#17-193-197) among the SpCas9 gRNA candidates were measured (FIGS. 20 to 22).

In all of the Huh7 and HepG2 cell lines, when a guide nucleic acid of the Sp#17+Sp#90+Sp#197 combination or the Sp#17+Sp#193+Sp#197 combination was used, rather than guide nucleic acids of Sp#17, Sp#90, Sp#193 and Sp#197 alone, it can be seen that the HBeAg and HBsAg inhibitory capacity was excellent.

### Result 3) Confirmation of HBeAg and HBsAg inhibitory capacity of HBV genotype A, HBV genotype B, HBV genotype C and HBV genotype D of SpCas9 gRNA candidates

In HBV genotype A, HBV genotype B, HBV genotype C and HBV genotype D cell lines prepared by introducing pAAV HBV genotype A, B, C and D into the human liver cancer cell line Huh7, respectively (respectively referred to as genotype A, genotype B, genotype C and genotype D), HBeAg and HBsAg levels of SpCas9 gRNA candidates Sp#19 3(named N#193), Sp#197 (named N#197), Sp#17+90+197 (named N#17+90+197) and Sp#17+193+197 (named N#17+193+197) were measured.

It can be seen that each of the SpCas9 gRNA candidates had different HBeAg and HBsAg levels in each of the HBV genotype A, HBV genotype B, HBV genotype C and HBV genotype D cell lines.

As comparing all of the results of the genotypes A, B, C and D, it was confirmed that N#193 and N#197 (single gRNA) tended to decrease HBeAg, but did not decrease HBsAg, but N#17-90-193 and N#17-193-197 effectively decreased HBeAg and HBsAg.

That is, it can be seen that N#17-90-193 and N#17-193-197 effectively inhibited HBV genotypes A, B, C and D.

### [Industrial Applicability]

The present application relates to a method for inhibiting the proliferation of various HBV genotypes.

## Claims

1. A method for inhibiting a Hepatitis B virus (HBV) proliferation, comprising introducing into a subject a composition comprising:
a) a guide nucleic acid, or a nucleic acid sequence encoding the same; and
b) a Cas protein, or a nucleic acid sequence encoding the same;
wherein the hepatitis B virus is at least two or more HBV genotypes selected from HBV type A, HBV type B, HBV type C, HBV type D, HBV type E, HBV type F and HBV type G,
wherein the guide nucleic acid selected from SEQ ID NO.51, 52, 56, 57, 69, 70, 73, 74, 75, 76, 80, 85, 87 and 89,
thereby inducing an indel in the nucleic acid sequence in the gene of the hepatitis B infection.

2. A method for inactivating a conserved sequence in a Hepatitis B virus (HBV) gene, comprising
introducing into a subject a composition comprising:
a) a guide nucleic acid, or a nucleic acid sequence encoding the same; and
b) a Cas protein, or a nucleic acid sequence encoding the same;
the guide nucleic acid is a sequence having a complementarity or homology with the conserved sequence in part or in whole,
wherein the guide nucleic acid selected from SEQ ID NO.51, 52, 56, 57, 69, 70, 73, 74, 75, 76, 80, 85, 87 and 89,
wherein the hepatitis B virus is at least two or more HBV genotypes selected from HBV type A, HBV type B, HBV type C, HBV type D, HBV type E, HBV type F and HBV type G,
wherein the conserved sequence is one or more selected from SEQ ID NO. 1 to 45 which is conserved in the HBV genotypes,
the inactivating is being cleaved the conserved sequence.

3. The method of claim 1 or claim 2,
wherein the Cas protein is one or more selected from Cas9 protein derived from Streptococcus pyogenes, Cas9 protein derived from Campylobacter jejuni.

4. The method of claim 1 or claim 2,
wherein the guide nucleic acid of the a) is one selected from SEQ ID NO.51, 52, 56, 57, 69, 70, 73, 74, 75 and 76,
the Cas9 of the b) is Cas9 protein derived from Streptococcus pyogenes.

5. The method of claim 1 or claim 2,
wherein the guide nucleic acid of the a) is one selected from SEQ ID NO.80, 85, 87 and 89,
the Cas9 of the b) is Cas9 protein derived from Campylobacter jejuni.

6. The method of claim 1 or claim 2,
wherein the guide nucleic acid and the Cas protein are introduced in the form of vector.

7. The method of claim 6,
wherein the vector is one or more vectors selected from the group consisting of retrovirus, lentivirus, adenovirus, adeno-associated virus (AAV), vaccinia virus, poxvirus and herpes simplex virus.

8. The method of claim 4,
wherein the guide nucleic acid is introduced with SEQ ID NO.51, 57 and 73.

9. The method of claim 4,
wherein the guide nucleic acid is introduced with SEQ ID NO.51, 73 and 76.

10. The method of claim 1 or claim 2,
wherein the method inhibits one or more selected from HBeAg and HBsAg which are antigens of the hepatitis B virus.

11. A composition for inhibiting a proliferation of a Hepatitis B virus targeting a conserved sequence in the hepatitis B virus (HBV) genome,
the composition comprises
a) a guide nucleic acid, or a nucleic acid sequence encoding the same; and
b) a Cas protein, or a nucleic acid sequence encoding the same;
wherein the guide nucleic acid selected from SEQ ID NO.51, 52, 56, 57, 69, 70, 73, 74, 75, 76, 80, 85, 87 and 89,
wherein the conserved sequence is one or more selected from SEQ ID NO. 1 to 45.

12. The composition of claim 11,
wherein the Cas protein is one or more selected from Cas9 protein derived from Streptococcus pyogenes, Cas9 protein derived from Campylobacter jejuni.

13. The composition of claim 11,
wherein the composition is in the form of vector.

14. The composition of claim 11,
wherein the guide nucleic acid of the a) is one selected from SEQ ID NO.51, 52, 56, 57, 69, 70, 73, 74, 75 and 76,
the Cas9 of the b) is Cas9 protein derived from Streptococcus pyogenes.

15. The composition of claim 11,
wherein the guide nucleic acid of the a) is one selected from SEQ ID NO. 80, 85, 87 and 89,
the Cas9 of the b) is Cas9 protein derived from Campylobacter jejuni.

16. The composition of claim 11,
wherein the composition comprises a guide nucleic acid comprising SEQ ID NO.51, 57 and 73.

17. The composition of claim 11,
wherein the composition comprises a guide nucleic acid comprising SEQ ID NO.51, 73 and 76.


<110>     TOOLGEN INCORPORATED

<120>     A Composition for inhibiting Hepatitis B virus proliferation and
          its method

<130>     OPP19-011-PCT

<150>     US 62/835628
<151>     2019-04-18

<160>     93

<170>     KoPatentIn 3.0

<210>     1
<211>     20
<212>     DNA
<213>     Artificial Sequence

<220>
<223>     Sp20-viHBV-B-#10


<400>     1
gtaacacgag caggggtcct                                                   20


<210>     2
<211>     20
<212>     DNA
<213>     Artificial Sequence

<220>
<223>     Sp20-viHBV-B-#11


<400>     2
ccccgcctgt aacacgagca                                                   20


<210>     3
<211>     20
<212>     DNA
<213>     Artificial Sequence

<220>
<223>     Sp20-viHBV-B-#12


<400>     3
accccgcctg taacacgagc                                                   20


<210>     4
<211>     20
<212>     DNA
<213>     Artificial Sequence

<220>
<223>     Sp20-viHBV-B-#13


<400>     4

aggaccccctg ctcgtgttac                                                    20


<210>    5
<211>    20
<212>    DNA
<213>    Artificial Sequence

<220>
<223>    Sp20-viHBV-B-#14


<400>    5
acccctgctc gtgttacagg                                                    20


<210>    6
<211>    20
<212>    DNA
<213>    Artificial Sequence

<220>
<223>    Sp20-viHBV-B-#17


<400>    6
caccacgagt ctagactctg                                                    20


<210>    7
<211>    20
<212>    DNA
<213>    Artificial Sequence

<220>
<223>    Sp20-viHBV-B-#20


<400>    7
ggacttctct caattttcta                                                    20


<210>    8
<211>    20
<212>    DNA
<213>    Artificial Sequence

<220>
<223>    Sp20-viHBV-B-#52


<400>    8
cctacgaacc actgaacaaa                                                    20


<210>    9
<211>    20
<212>    DNA
<213>    Artificial Sequence

<220>
<223>    Sp20-viHBV-B-#53

<400>    9
ccatttgttc agtggttcgt                                                    20


<210>    10
<211>    20
<212>    DNA
<213>    Artificial Sequence

<220>
<223>    Sp20-viHBV-B-#54


<400>    10
catttgttca gtggttcgta                                                    20


<210>    11
<211>    20
<212>    DNA
<213>    Artificial Sequence

<220>
<223>    Sp20-viHBV-B-#89


<400>    11
gggttgcgtc agcaaacact                                                    20


<210>    12
<211>    20
<212>    DNA
<213>    Artificial Sequence

<220>
<223>    Sp20-viHBV-B-#90


<400>    12
tttgctgacg caacccccac                                                    20


<210>    13
<211>    20
<212>    DNA
<213>    Artificial Sequence

<220>
<223>    Sp20-viHBV-B-#101


<400>    13
tccgcagtat ggatcggcag                                                    20


<210>    14
<211>    20
<212>    DNA
<213>    Artificial Sequence

<220>

<223>    Sp20-viHBV-B-#102

<400>    14
aggagttccg cagtatggat                                                    20

<210>    15
<211>    20
<212>    DNA
<213>    Artificial Sequence

<220>
<223>    Sp20-viHBV-B-#103

<400>    15
tcctctgccg atccatactg                                                    20

<210>    16
<211>    20
<212>    DNA
<213>    Artificial Sequence

<220>
<223>    Sp20-viHBV-B-#113

<400>    16
cgtcccgcgc aggatccagt                                                    20

<210>    17
<211>    20
<212>    DNA
<213>    Artificial Sequence

<220>
<223>    Sp20-viHBV-B-#117

<400>    17
ccgcgggatt cagcgccgac                                                    20

<210>    18
<211>    20
<212>    DNA
<213>    Artificial Sequence

<220>
<223>    Sp20-viHBV-B-#118

<400>    18
tccgcgggat tcagcgccga                                                    20

<210>    19
<211>    20
<212>    DNA
<213>    Artificial Sequence

<220>
<223>    Sp20-viHBV-B-#119


<400>    19
cccgtcggcg ctgaatcccg                                                    20


<210>    20
<211>    20
<212>    DNA
<213>    Artificial Sequence

<220>
<223>    Sp20-viHBV-B-#138


<400>    20
gtaaagagag gtgcgccccg                                                    20


<210>    21
<211>    20
<212>    DNA
<213>    Artificial Sequence

<220>
<223>    Sp20-viHBV-B-#140


<400>    21
ggggcgcacc tctctttacg                                                    20


<210>    22
<211>    20
<212>    DNA
<213>    Artificial Sequence

<220>
<223>    Sp20-viHBV-B-#142


<400>    22
gaagcgaagt gcacacggtc                                                    20


<210>    23
<211>    20
<212>    DNA
<213>    Artificial Sequence

<220>
<223>    Sp20-viHBV-B-#143


<400>    23
ggtctccatg cgacgtgcag                                                    20


<210>    24
<211>    20

<212>    DNA
<213>    Artificial Sequence

<220>
<223>    Sp20-viHBV-B-#154


<400>    24
aatgtcaacg accgaccttg                                                    20


<210>    25
<211>    20
<212>    DNA
<213>    Artificial Sequence

<220>
<223>    Sp20-viHBV-B-#159


<400>    25
aggaggctgt aggcataaat                                                    20


<210>    26
<211>    20
<212>    DNA
<213>    Artificial Sequence

<220>
<223>    Sp20-viHBV-B-#186


<400>    26
cggaagtgtt gataagatag                                                    20


<210>    27
<211>    20
<212>    DNA
<213>    Artificial Sequence

<220>
<223>    Sp20-viHBV-B-#187


<400>    27
ccggaagtgt tgataagata                                                    20


<210>    28
<211>    20
<212>    DNA
<213>    Artificial Sequence

<220>
<223>    Sp20-viHBV-B-#193


<400>    28
gcgagggagt tcttcttcta                                                    20

<210>    29
<211>    20
<212>    DNA
<213>    Artificial Sequence

<220>
<223>    Sp20-viHBV-B-#194


<400>    29
gaccttcgtc tgcgaggcga                                                    20


<210>    30
<211>    20
<212>    DNA
<213>    Artificial Sequence

<220>
<223>    Sp20-viHBV-B-#196


<400>    30
gattgagacc ttcgtctgcg                                                    20


<210>    31
<211>    20
<212>    DNA
<213>    Artificial Sequence

<220>
<223>    Sp20-viHBV-B-#197


<400>    31
ctccctcgcc tcgcagacga                                                    20


<210>    32
<211>    20
<212>    DNA
<213>    Artificial Sequence

<220>
<223>    Sp20-viHBV-B-#198


<400>    32
gattgagatc ttctgcgacg                                                    20


<210>    33
<211>    20
<212>    DNA
<213>    Artificial Sequence

<220>
<223>    Sp20-viHBV-B-#199


<400>    33
gtcgcagaag atctcaatct                                                    20

<210>	34
<211>	20
<212>	DNA
<213>	Artificial Sequence

<220>
<223>	Sp20-viHBV-B-#200


<400>	34
tcgcagaaga tctcaatctc	20


<210>	35
<211>	22
<212>	DNA
<213>	Artificial Sequence

<220>
<223>	Cj22-viHBV-B-#06


<400>	35
tgtcaacaag aaaaaccccg cc	22


<210>	36
<211>	22
<212>	DNA
<213>	Artificial Sequence

<220>
<223>	Cj22-viHBV-B-#20


<400>	36
aagccctacg aaccactgaa ca	22


<210>	37
<211>	22
<212>	DNA
<213>	Artificial Sequence

<220>
<223>	Cj22-viHBV-B-#23


<400>	37
ttaccaattt tcttttgtct tt	22


<210>	38
<211>	22
<212>	DNA
<213>	Artificial Sequence

<220>
<223>	Cj22-viHBV-B-#40

<400>    38
acgtcccgcg caggatccag tt                                                22


<210>    39
<211>    22
<212>    DNA
<213>    Artificial Sequence

<220>
<223>    Cj22-viHBV-B-#44


<400>    39
gtgcacacgg tccggcagat ga                                                22


<210>    40
<211>    22
<212>    DNA
<213>    Artificial Sequence

<220>
<223>    Cj22-viHBV-B-#45


<400>    40
gtgccttctc atctgccgga cc                                                22


<210>    41
<211>    22
<212>    DNA
<213>    Artificial Sequence

<220>
<223>    Cj22-viHBV-B-#46


<400>    41
cgacgtgcag aggtgaagcg aa                                                22


<210>    42
<211>    22
<212>    DNA
<213>    Artificial Sequence

<220>
<223>    Cj22-viHBV-B-#47


<400>    42
tgcgacgtgc agaggtgaag cg                                                22


<210>    43
<211>    22
<212>    DNA
<213>    Artificial Sequence

<220>
<223>    Cj22-viHBV-B-#48

<400>    43
gaccgtgtgc acttcgcttc ac                                                22

<210>    44
<211>    22
<212>    DNA
<213>    Artificial Sequence

<220>
<223>    Cj22-viHBV-B-#57

<400>    44
atgtccatgc cccaaagcca cc                                                22

<210>    45
<211>    22
<212>    DNA
<213>    Artificial Sequence

<220>
<223>    Cj22-viHBV-B-#67

<400>    45
gaccaccaaa tgcccctatc tt                                                22

<210>    46
<211>    20
<212>    RNA
<213>    Artificial Sequence

<220>
<223>    Sp20-viHBV-B-#10G

<400>    46
guaacacgag caggggnccu                                                   20

<210>    47
<211>    20
<212>    RNA
<213>    Artificial Sequence

<220>
<223>    Sp20-viHBV-B-#11G

<400>    47
ccccgccugu aacacgagca                                                   20

<210>    48
<211>    20
<212>    RNA
<213>    Artificial Sequence

<220>
<223>    Sp20-viHBV-B-#12G


<400>    48
accccgccug uaacacgagc                                                    20


<210>    49
<211>    20
<212>    RNA
<213>    Artificial Sequence

<220>
<223>    Sp20-viHBV-B-#13G


<400>    49
aggaccccug cucguguuac                                                    20


<210>    50
<211>    20
<212>    RNA
<213>    Artificial Sequence

<220>
<223>    Sp20-viHBV-B-#14G


<400>    50
accccugcuc guguuacagg                                                    20


<210>    51
<211>    20
<212>    RNA
<213>    Artificial Sequence

<220>
<223>    Sp20-viHBV-B-#17G


<400>    51
caccacgagu cuagacucug                                                    20


<210>    52
<211>    20
<212>    RNA
<213>    Artificial Sequence

<220>
<223>    Sp20-viHBV-B-#20G


<400>    52
ggacuucucu caauuuucua                                                    20


<210>    53
<211>    20
<212>    RNA

<213>    Artificial Sequence

<220>
<223>    Sp20-viHBV-B-#52G


<400>    53
ccuacgaacc acugaacaaa                                                    20


<210>    54
<211>    20
<212>    RNA
<213>    Artificial Sequence

<220>
<223>    Sp20-viHBV-B-#53G


<400>    54
ccauuuguuc agugguucgu                                                    20


<210>    55
<211>    20
<212>    RNA
<213>    Artificial Sequence

<220>
<223>    Sp20-viHBV-B-#54G


<400>    55
cauuuguuca gugguucgua                                                    20


<210>    56
<211>    20
<212>    RNA
<213>    Artificial Sequence

<220>
<223>    Sp20-viHBV-B-#89G


<400>    56
ggguugcguc agcaaacacu                                                    20


<210>    57
<211>    20
<212>    RNA
<213>    Artificial Sequence

<220>
<223>    Sp20-viHBV-B-#90G


<400>    57
uuugcugacg caacccccac                                                    20


<210>    58

<211>    20
<212>    RNA
<213>    Artificial Sequence

<220>
<223>    Sp20-viHBV-B-#101G


<400>    58
uccgcaguau ggaucggcag                                                    20


<210>    59
<211>    20
<212>    RNA
<213>    Artificial Sequence

<220>
<223>    Sp20-viHBV-B-#102G


<400>    59
aggaguuccg caguauggau                                                    20


<210>    60
<211>    20
<212>    RNA
<213>    Artificial Sequence

<220>
<223>    Sp20-viHBV-B-#103G


<400>    60
uccucugccg auccauacug                                                    20


<210>    61
<211>    20
<212>    RNA
<213>    Artificial Sequence

<220>
<223>    Sp20-viHBV-B-#113G


<400>    61
cgucccgcgc aggauccagu                                                    20


<210>    62
<211>    20
<212>    RNA
<213>    Artificial Sequence

<220>
<223>    Sp20-viHBV-B-#117G


<400>    62
ccgcgggauu cagcgccgac                                                    20

<210>    63
<211>    20
<212>    RNA
<213>    Artificial Sequence

<220>
<223>    Sp20-viHBV-B-#118G


<400>    63
uccgcgggau ucagcgccga                                                    20


<210>    64
<211>    20
<212>    RNA
<213>    Artificial Sequence

<220>
<223>    Sp20-viHBV-B-#119G


<400>    64
cccgucggcg cugaaucccg                                                    20


<210>    65
<211>    20
<212>    RNA
<213>    Artificial Sequence

<220>
<223>    Sp20-viHBV-B-#138G


<400>    65
guaaagagag gugcgccccg                                                    20


<210>    66
<211>    20
<212>    RNA
<213>    Artificial Sequence

<220>
<223>    Sp20-viHBV-B-#140G


<400>    66
ggggcgcacc ucucuuuacg                                                    20


<210>    67
<211>    20
<212>    RNA
<213>    Artificial Sequence

<220>
<223>    Sp20-viHBV-B-#142G


<400>    67

gaagcgaagu gcacacgguc                                                    20

<210>    68
<211>    20
<212>    RNA
<213>    Artificial Sequence

<220>
<223>    Sp20-viHBV-B-#143G

<400>    68
ggucuccaug cgacgugcag                                                    20

<210>    69
<211>    20
<212>    RNA
<213>    Artificial Sequence

<220>
<223>    Sp20-viHBV-B-#154G

<400>    69
aaugucaacg accgaccuug                                                    20

<210>    70
<211>    20
<212>    RNA
<213>    Artificial Sequence

<220>
<223>    Sp20-viHBV-B-#159G

<400>    70
aggaggcugu aggcauaaau                                                    20

<210>    71
<211>    20
<212>    RNA
<213>    Artificial Sequence

<220>
<223>    Sp20-viHBV-B-#186G

<400>    71
cggaaguguu gauaagauag                                                    20

<210>    72
<211>    20
<212>    RNA
<213>    Artificial Sequence

<220>
<223>    Sp20-viHBV-B-#187G

<400>    72
ccggaagugu ugauaagaua                                                    20

<210>    73
<211>    20
<212>    RNA
<213>    Artificial Sequence

<220>
<223>    Sp20-viHBV-B-#193G

<400>    73
gcgagggagu ucuucuucua                                                    20

<210>    74
<211>    20
<212>    RNA
<213>    Artificial Sequence

<220>
<223>    Sp20-viHBV-B-#194G

<400>    74
gaccuucguc ugcgaggcga                                                    20

<210>    75
<211>    20
<212>    RNA
<213>    Artificial Sequence

<220>
<223>    Sp20-viHBV-B-#196G

<400>    75
gauugagacc uucgucugcg                                                    20

<210>    76
<211>    20
<212>    RNA
<213>    Artificial Sequence

<220>
<223>    Sp20-viHBV-B-#197G

<400>    76
   cucccucgcc ucgcagacga                                                   20

<210>    77
<211>    20
<212>    RNA
<213>    Artificial Sequence

<220>

<223>    Sp20-viHBV-B-#198G

<400>    77
gauugagauc uucugcgacg                                                    20

<210>    78
<211>    20
<212>    RNA
<213>    Artificial Sequence

<220>
<223>    Sp20-viHBV-B-#199G

<400>    78
gucgcagaag aucucaaucu                                                    20

<210>    79
<211>    20
<212>    RNA
<213>    Artificial Sequence

<220>
<223>    Sp20-viHBV-B-#200G

<400>    79
ucgcagaaga ucucaaucuc                                                    20

<210>    80
<211>    22
<212>    RNA
<213>    Artificial Sequence

<220>
<223>    Cj22-viHBV-B-#06G

<400>    80
ugucaacaag aaaaaccccg cc                                                 22

<210>    81
<211>    22
<212>    RNA
<213>    Artificial Sequence

<220>
<223>    Cj22-viHBV-B-#20G

<400>    81
aagcccuacg aaccacugaa ca                                                 22

<210>    82
<211>    22
<212>    RNA
<213>    Artificial Sequence

<220>
<223>    Cj22-viHBV-B-#23G


<400>    82
uuaccaauuu ucuuuugucu uu                                                22


<210>    83
<211>    22
<212>    RNA
<213>    Artificial Sequence

<220>
<223>    Cj22-viHBV-B-#40G


<400>    83
acgucccgcg caggauccag uu                                                22


<210>    84
<211>    22
<212>    RNA
<213>    Artificial Sequence

<220>
<223>    Cj22-viHBV-B-#44G


<400>    84
gugcacacgg uccggcagau ga                                                22


<210>    85
<211>    22
<212>    RNA
<213>    Artificial Sequence

<220>
<223>    Cj22-viHBV-B-#45G


<400>    85
gugccuucuc aucugccgga cc                                                22


<210>    86
<211>    22
<212>    RNA
<213>    Artificial Sequence

<220>
<223>    Cj22-viHBV-B-#46G


<400>    86
cgacgugcag aggugaagcg aa                                                22


<210>    87
<211>    22

<212>    RNA
<213>    Artificial Sequence

<220>
<223>    Cj22-viHBV-B-#47G


<400>    87
ugcgacgugc agaggugaag cg                                              22


<210>    88
<211>    22
<212>    RNA
<213>    Artificial Sequence

<220>
<223>    Cj22-viHBV-B-#48G


<400>    88
gaccgugugc acuucgcuuc ac                                              22


<210>    89
<211>    22
<212>    RNA
<213>    Artificial Sequence

<220>
<223>    Cj22-viHBV-B-#57G


<400>    89
auguccaugc cccaaagcca cc                                              22


<210>    90
<211>    22
<212>    RNA
<213>    Artificial Sequence

<220>
<223>    Cj22-viHBV-B-#67G


<400>    90
gaccaccaaa ugccccuauc uu                                              22


<210>    91
<211>    3215
<212>    DNA
<213>    Artificial Sequence

<220>
<223>    virtual HBV B genotype sequences


<400>    91
ctccaccact ttccaccaaa ctcttcaaga tcccagagtc agggccctgt actttcctgc      60

tggtggctcc agttcaggaa cagtgagccc tgctcagaat actgtctctg ccatatcgtc     120

aatcttatcg aagactgggg accctgtacc gaacatggag aacatcgcat caggactcct 180

aggacccctg ctcgtgttac aggcggggtt tttcttgttg acaaaaatcc tcacaatacc 240

acagagtcta gactcgtggt ggacttctct caattttcta gggggaacac ccgtgtgtct 300

tggccaaaat tcgcagtccc aaatctccag tcactcacca acctgttgtc ctccaatttg 360

tcctggttat cgctggatgt gtctgcggcg ttttatcatc ttcctctgca tcctgctgct 420

atgcctcatc ttcttgttgg ttcttctgga ctatcaaggt atgttgcccg tttgtcctct 480

aattccagga tcatcaacaa ccagcaccgg accatgcaaa acctgcacaa ctcctgctca 540

aggaacctct atgtttccct catgttgctg tacaaaacct acggacggaa actgcacctg 600

tattcccatc ccatcatctt gggctttcgc aaaataccta tgggagtggg cctcagtccg 660

tttctcttgg ctcagtttac tagtgccatt tgttcagtgg ttcgtagggc tttcccccac 720

tgtctggctt tcagttatat ggatgatgtg gttttggggg ccaagtctgt acaacatctt 780

gagtcccttt atgccgctgt taccaatttt cttttgtctt tgggtataca tttaaaccct 840

cacaaaacaa aaagatgggg atattccctt aacttcatgg gatatgtaat tgggagttgg 900

ggcacattgc cacaggaaca tattgtacaa aaaatcaaaa tgtgttttag gaaacttcct 960

gtaaacaggc ctattgattg gaaagtatgt caacgaattg tgggtctttt ggggtttgcc 1020

gcccctttca cgcaatgtgg atatcctgct ttaatgcctt tatatgcatg tatacaagca 1080

aaacaggctt ttactttctc gccaacttac aaggcctttc taagtaaaca gtatctgaac 1140

ctttaccccg ttgctcggca acggcctggt ctgtgccaag tgtttgctga cgcaaccccc 1200

actggttggg gcttggccat aggccatcag cgcatgcgtg gaacctttgt gtctcctctg 1260

ccgatccata ctgcggaact cctagccgct tgttttgctc gcagcaggtc tggggcaaaa 1320

ctcatcggga ctgacaattc tgtcgtgctc tcccgcaagt atacatcatt tccatggctg 1380

ctaggctgtg ctgccaactg gatcctgcgc gggacgtcct ttgtttacgt cccgtcggcg 1440

ctgaatcccg cggacgaccc ctcccggggc cgcttggggc tctaccgccc gcttctccgc 1500

ctgttgtacc gaccgaccac ggggcgcacc tctctttacg cggactcccc gtctgtgcct 1560

tctcatctgc cggaccgtgt gcacttcgct tcacctctgc acgtcgcatg gagaccaccg 1620

tgaacgccca ccggaacctg cccaaggtct tgcataagag gactcttgga ctttcagcaa 1680

tgtcaacgac cgaccttgag gcatacttca aagactgtgt gtttactgag tgggaggagt 1740

tgggggagga gattaggtta aaggtctttg tactaggagg ctgtaggcat aaattggtgt 1800

gttcaccagc accatgcaac tttttcacct ctgcctaatc atctcatgtt catgtcctac 1860

tgttcaagcc tccaagctgt gccttgggtg gctttggggc atggacattg acccgtataa 1920

agaatttgga gcttctgtgg agttactctc ttttttgcct tctgacttct ttccttctat 1980

tcgagatctc ctcgacaccg cctctgctct gtatcgggag gccttagagt ctccggaaca 2040

ttgttcacct caccatacgg cactcaggca agctattctg tgttggggtg agttgatgaa 2100

tctagccacc tgggtgggaa gtaatttgga agatccagca tccagggaat tagtagtcag 2160

ctatgtcaac gttaatatgg gcctaaaaat cagacaacta ttgtggtttc acatttcctg 2220

tcttactttt gggagagaaa ctgttcttga atatttggtg tcttttggag tgtggattcg 2280

cactcctcct gcatatagac caccaaatgc ccctatctta tcaacacttc cggaaactac 2340

tgttgttaga cgaagaggca ggtcccctag aagaagaact ccctcgcctc gcagacgaag 2400

gtctcaatcg ccgcgtcgca gaagatctca atctcgggaa tctcaatgtt agtattcctt 2460

ggacacataa ggtgggaaac tttacggggc tttattcttc tacggtacct tgctttaatc 2520

ctaaatggca aactccttct tttcctgaca ttcatttgca ggaggacatt gttgatagat 2580

gtaagcaatt tgtgggcccc cttacagtaa atgaaaacag gagactaaaa ttaattatgc 2640

ctgctaggtt ttatcccaat gttactaaat atttgccctt agataaaggg atcaaaccgt 2700

attatccaga gcatgtagtt aatcattact tccagacgag acattattta cacactcttt 2760

ggaaggcggg tatcttatat aaaagagagt ccacacgtag cgcctcattt tgcgggtcac 2820

catattcttg ggaacaagat ctacagcatg ggaggttggt cttccaaacc tcgaaaaggc 2880

atggggacaa atctttctgt ccccaatccc ctgggattct tccccgatca tcagttggac 2940

cctgcattca aagccaactc agaaaatcca gattgggacc tcaacccgca caaggacaac 3000

tggccggacg ccaacaaggt gggagtggga gcattcgggc cagggttcac ccctccccat 3060

gggggactgt tggggtggag ccctcaggct cagggcatac tcacaactgt gccagcagct 3120

cctcctcctg cctccaccaa tcggcagtca ggaaggcagc ctactccctt atctccacct 3180

ctaagggaca ctcatcctca ggccatgcag tggaa 3215

<210> 92
<211> 3215
<212> DNA
<213> Artificial Sequence

<220>
<223> virtual HBV C genotype sequences

<400> 92
ctccacaaca ttccaccaag ctctgctaga tcccagagtg aggggcctat actttcctgc 60

tggtggctcc agttccggaa cagtaaaccc tgttccgact actgcctcac ccatatcgtc 120

aatcttctcg aggactgggg accctgcacc gaacatggag aacacaacat caggattcct 180

aggacccctg ctcgtgttac aggcggggtt tttcttgttg acaagaatcc tcacaatacc 240

acagagtcta gactcgtggt ggacttctct caattttcta gggggagcac ccacgtgtcc 300

tggccaaaat tcgcagtccc caacctccaa tcactcacca acctcttgtc ctccaatttg 360

tcctggctat cgctggatgt gtctgcggcg ttttatcata ttcctcttca tcctgctgct 420

atgcctcatc ttcttgttgg ttcttctgga ctaccaaggt atgttgcccg tttgtcctct 480

acttccagga acatcaacta ccagcacggg accatgcaag acctgcacga ttcctgctca 540

aggaacctct atgtttccct cttgttgctg tacaaaacct tcggacggaa actgcacttg 600

tattcccatc ccatcatcct gggctttcgc aagattccta tgggagtggg cctcagtccg 660

tttctcctgg ctcagtttac tagtgccatt tgttcagtgg ttcgtagggc tttcccccac 720

tgtttggctt tcagttatat ggatgatgtg gtattggggg ccaagtctgt acaacatctt 780

gagtcccttt ttacctctat taccaatttt cttttgtctt tgggtataca tttgaaccct 840

aataaaacca aacgttgggg ctactccctt aacttcatgg gatatgtaat tggaagttgg 900

ggtactttac cacaggaaca tattgtacta aaaatcaagc aatgttttcg aaaactgcct 960

gtaaatagac ctattgattg gaaagtatgt caaagaattg tgggtctttt gggctttgct 1020

gcccctttta cacaatgtgg ctatcctgcc ttaatgcctt tatatgcatg tatacaatct 1080

aagcaggctt tcactttctc gccaacttac aaggcctttc tgtgtaaaca atatctgaac 1140

ctttaccccg ttgcccggca acggtcaggt ctctgccaag tgtttgctga cgcaaccccc 1200

actggatggg gcttggccat aggccatcgg cgcatgcgtg gaacctttgt ggctcctctg 1260

ccgatccata ctgcggaact cctagcagct tgttttgctc gcagccggtc tggagcgaaa 1320

cttatcggaa ccgacaactc tgttgtcctc tctcggaaat acacctcctt tccatggctg 1380

ctagggtgtg ctgccaactg gatcctgcgc gggacgtcct ttgtctacgt cccgtcggcg 1440

ctgaatcccg cggacgaccc gtctcggggc cgtttgggac tctaccgtcc ccttcttcat 1500

ctgccgttcc ggccgaccac ggggcgcacc tctctttacg cggtctcccc gtctgtgcct 1560

tctcatctgc cggaccgtgt gcacttcgct tcacctctgc acgtcgcatg gagaccaccg 1620

tgaacgccca ccaggtcttg cccaaggtct tacataagag gactcttgga ctctcagcaa 1680

tgtcaacgac cgaccttgag gcatacttca aagactgttt gtttaaagac tgggaggagt 1740

tgggggagga gattaggtta aagatctttg tactaggagg ctgtaggcat aaattggtct 1800

gttcaccagc accatgcaac tttttcacct ctgcctaatc atctcatgtt catgtcctac 1860

tgttcaagcc tccaagctgt gccttgggtg gctttggggc atggacattg acccgtataa 1920

agaatttgga gcttctgtgg agttactctc ttttttgcct tctgacttct ttccttctat 1980

tcgagatctc ctcgacaccg cctctgctct gtatcgggag gccttagagt ctccggaaca 2040

ttgttcacct caccatacag cactcaggca agctattctg tgttggggtg agttgatgaa 2100

tctggccacc tgggtgggaa gtaatttgga agacccagca tccagggaat tagtagtcag 2160

ctatgtcaat gttaatatgg gcctaaaaat cagacaacta ttgtggtttc acatttcctg 2220

tcttactttt ggaagagaaa ctgttcttga gtatttggtg tcttttggag tgtggattcg 2280

cactcctccc gcttacagac caccaaatgc ccctatctta tcaacacttc cggaaactac 2340

tgttgttaga cgacgaggca ggtcccctag aagaagaact ccctcgcctc gcagacgaag 2400

gtctcaatcg ccgcgtcgca gaagatctca atctcgggaa tctcaatgtt agtatccctt 2460

ggactcataa ggtgggaaac tttactgggc tttattcttc tactgtacct gtctttaatc 2520

ctgagtggca aactccctcc tttcctcaca ttcatttaca ggaggacatt attaatagat 2580

gtcaacaata tgtgggccct cttacagtta atgaaaaaag gagattaaaa ttaattatgc 2640

ctgctaggtt ctatcctaac cttaccaaat atttgccctt ggacaaaggc attaaacctt 2700

attatcctga acatgcagtt aatcattact tcaaaactag gcattattta catactctgt 2760

ggaaggctgg cattctatat aagagagaaa ctacacgcag cgcctcattt tgtgggtcac 2820

catattcttg ggaacaagag ctacagcatg ggaggttggt cttccaaacc tcgacaaggc 2880

atggggacga atctttctgt tcccaatcct ctgggattct ttcccgatca ccagttggac 2940

cctgcgttcg gagccaactc aaacaatcca gattgggact tcaaccccaa caaggatcac 3000

tggccagagg caaatcaggt aggagcggga gcattcgggc cagggttcac cccaccacac 3060

ggcggtcttt tggggtggag ccctcaggct cagggcatat tgacaacagt gccagcagca 3120

cctcctcctg cctccaccaa tcggcagtca ggaagacagc ctactcccat ctctccacct 3180

ctaagagaca gtcatcctca ggccatgcag tggaa 3215

<210> 93
<211> 3182
<212> DNA
<213> Artificial Sequence

<220>
<223> virtual HBV D genotype sequences

<400> 93
ctccacaacc ttccaccaaa ctctgcaaga tcccagagtg agaggcctgt atttccctgc 60

tggtggctcc agttcaggaa cagtaaaccc tgttccgact actgtctctc ccatatcgtc 120

aatcttctcg aggattgggg accctgcgct gaacatggag aacatcacat caggattcct 180

aggacccctg ctcgtgttac aggcggggtt tttcttgttg acaagaatcc tcacaatacc 240

gcagagtcta gactcgtggt ggacttctct caattttcta gggggaacta ccgtgtgtct 300

tggccaaaat tcgcagtccc caacctccaa tcactcacca acctcctgtc ctccaacttg 360

tcctggttat cgctggatgt gtctgcggcg ttttatcatc ttcctcttca tcctgctgct 420

atgcctcatc ttcttgttgg ttcttctgga ctatcaaggt atgttgcccg tttgtcctct 480

aattccagga tcttcaacca ccagcacggg accatgcaga acctgcacga ctcctgctca 540

aggaacctct atgtatccct cctgttgctg taccaaacct tcggacggaa attgcacctg 600

tattcccatc ccatcatcct gggctttcgg aaaattccta tgggagtggg cctcagcccg 660

tttctcctgg ctcagtttac tagtgccatt tgttcagtgg ttcgtagggc tttcccccac 720

tgtttggctt tcagttatat ggatgatgtg gtattggggg ccaagtctgt acagcatctt 780

gagtcccttt ttaccgctgt taccaatttt cttttgtctt tgggtataca tttaaaccct 840

aacaaaacaa aaagatgggg ttactcttta catttcatgg gctatgtcat tggatgttat 900

gggtcattgc cacaagatca catcatacag aaaatcaaag aatgttttag aaaacttcct 960

gttaacaggc ctattgattg gaaagtctgt caacgtattg tgggtctttt gggttttgct 1020

gcccctttta cacaatgtgg ttatcctgct ttaatgccct tgtatgcatg tattcaatct 1080

aagcaggctt tcactttctc gccaacttac aaggcctttc tgtgtaaaca atacctgaac 1140

ctttaccccg ttgcccggca acggccaggt ctgtgccaag tgtttgctga cgcaaccccc 1200

actggctggg gcttggtcat gggccatcag cgcatgcgtg gaacctttct ggctcctctg 1260

ccgatccata ctgcggaact cctagccgct tgttttgctc gcagcaggtc tggagcaaac 1320

attctcggga cggataactc tgttgttctc tcccgcaaat atacatcgtt tccatggctg 1380

ctaggctgtg ctgccaactg gatcctgcgc gggacgtcct ttgtttacgt cccgtcggcg 1440

ctgaatcccg cggacgaccc ttctcggggc cgcttgggac tctctcgtcc ccttctccgt 1500

ctgccgtttc gaccgaccac ggggcgcacc tctctttacg cggactcccc gtctgtgcct 1560

tctcatctgc cggaccgtgt gcacttcgct tcacctctgc acgtcgcatg gagaccaccg 1620

tgaacgccca ccaattcttg cccaaggtct tacataagag gactcttgga ctctctgtaa 1680

tgtcaacgac cgaccttgag gcatacttca aagactgttt gtttaaagac tgggaggagt 1740

tgggggagga gattagatta aaggtctttg tactaggagg ctgtaggcat aaattggtct 1800

gcgcaccagc accatgcaac tttttcacct ctgcctaatc atctcttgtt catgtcctac 1860

tgttcaagcc tccaagctgt gccttgggtg gctttggggc atggacattg acccttataa 1920

agaatttgga gctactgtgg agttactctc gtttttgcct tctgacttct ttccttcagt 1980

acgagatctt ctagataccg cctcagctct gtatcgggaa gccttagagt ctcctgagca 2040

ttgttcacct caccatactg cactcaggca agcaattctt tgctgggggg aactaatgac 2100

tctagctacc tgggtgggtg gtaatttgga agatccagca tccagggacc tagtagtcag 2160

ttatgtcaac actaatatgg gcctaaagtt caggcaacta ttgtggtttc acatttcttg 2220

tctcactttt ggaagagaaa cggtcataga gtatttggtg tctttcggag tgtggattcg 2280

cactcctcca gcttatagac caccaaatgc ccctatctta tcaacacttc cggagactac 2340

tgttgttaga cgacgaggca ggtcccctag aagaagaact ccctcgcctc gcagacgaag 2400

gtctcaatcg ccgcgtcgca gaagatctca atctcgggaa tctcaatgtt agtattcctt 2460

ggactcataa ggtgggaaac tttacggggc tttattcttc tactgtacct gtctttaacc 2520

ctcattggaa aacaccctct tttcctaata tacatttaca ccaagacatt atcaaaaaat 2580

gtgaacaatt tgtaggccca ctcacagtca atgagaaaag aagactgcaa ttgattatgc 2640

ctgctaggtt ttatccaaat gttaccaaat atttgccatt ggataagggt attaaacctt 2700

attatccaga acatctagtt aatcattact tccaaaccag acattattta cacactctat 2760

ggaaggcggg tatattatat aagagagaaa caacacatag cgcctcattt tgtgggtcac 2820

catattcttg ggaacaagag ctacagcatg gggcagaatc tttccaccag caatcctctg 2880

ggattctttc ccgaccacca gttggatcca gccttcagag caaacaccgc aaatccagat 2940

tgggacttca atcccaacaa ggacacctgg ccagacgcca acaaggtagg agctggagca 3000

ttcgggctgg gattcacccc accgcacgga ggccttttgg ggtggagccc tcaggctcag 3060

ggcatactac aaaccttgcc agcaaatccg cctcctgcct ctaccaatcg ccagtcagga 3120

aggcagccta ccccgctgtc tccacctttg agaaacactc atcctcaggc catgcagtgg 3180

aa 3182